# FASCICULE DE BREVET EUROPEEN

(11) **EP 1 948 231 B1**
(45) Date de publication et mention de la délivrance du brevet: **15.12.2010**
(21) Numéro de dépôt: 06820300.9
(22) Date de dépôt: 17.10.2006
(51) Int. Cl.: A61K 39/21, A61K 47/34, A61K 47/48, A61K 39/00

(54) **COMPOSITION COMPRENANT UN VECTEUR SYNTHÉTIQUE COLLOIDAL BIORÉSORBABLE ET UN VECTEUR VIRAL**
ZUSAMMENSETZUNG MIT EINEM KOLLOIDALEN SYNTHETISCHEN BIORESORBIERBAREN VEKTOR UND EINEM VIRALEN VEKTOR
COMPOSITION COMPRISING A COLLOIDAL SYNTHETIC BIORESORBABLE VECTOR AND A VIRAL VECTOR

(30) Priorité: 14.11.2005 FR 0553445
(43) Date de publication de la demande: 30.07.2008
(73) Titulaire: CENTRE NATIONAL DE LA RECHERCHE SCIENTIFIQUE (CNRS), 75016 Paris (FR)
(72) Inventeur: ATAMAN-ONAL, Yasemin, F-69003 Lyon (FR); DELAIR, Thierry, F-69700 Echalas (FR); VERRIER, Bernard, F-69440 Mornant (FR)
(74) Mandataire: Colombet, Alain André
(86) Numéro de dépôt international: PCT/FR2006/051039
(87) Numéro de publication internationale: WO 2007/057582

(56) Documents cités:
- WO-A-2005/027871
- SHARPE S ET AL: "Mucosal immunization with PLGA-microencapsulated DNA primes a SIV-specific CTL response revealed by boosting with cognate recombinant modified vaccinia virus Ankara" VIROLOGY, ACADEMIC PRESS,ORLANDO, US, vol. 313, no. 1, 15 août 2003 (2003-08-15), pages 13-21, XP004452386 ISSN: 0042-6822 cité dans la demande
- GANDER B: "Trends in particulate antigen and DNA delivery systems for vaccines" ADVANCED DRUG DELIVERY REVIEWS, AMSTERDAM, NL, vol. 57, no. 3, 10 janvier 2005 (2005-01-10), pages 321-323, XP004653207 ISSN: 0169-409X
- "Biodegradable microspheres prolong antigen presentation by macrophages" NEWSRX -- VACCINE WEEKLY, [Online] 22 mars 2006 (2006-03-22), XP002390751 Extrait de l'Internet: URL:http://www.newsrx.com/newsletters/Vacc ine-Weekly/2006-03-22/0322200633340VW.html > [extrait le 2006-07-17]

## Description

La présente invention concerne le domaine de la vaccination prophylactique et thérapeutique. En particulier, la présente invention a pour objet une composition vaccinale comprenant un vecteur viral permettant d'exprimer des antigènes protéques et un vecteur synthétique exprimant des antigènes protéiques.

Historiquement, les antigènes utilisés dans les vaccins sont, soit des microorganismes vivants dont le pouvoir pathogène a été atténué, soit des microorganismes tués. Cependant ces vaccins provoquent souvent des effets secondaires non désirables lors de l'administration et dans de rares cas des complications très graves. Ainsi, depuis de nombreuses années, pour assurer la sécurité vaccinale, on privilégie l'utilisation des fractions purifiées des microorganismes dans les vaccins. Un tel immunogène, administré seul, peut ne pas induire de réponse immune suffisante. Selon l'art antérieur, de nombreuses stratégies vaccinales ont été développées pour augmenter ou orienter la réponse immunitaire induite par les immunogènes purifiés présents dans un vaccin.

La stratégie la plus directe consiste à associer la substance protéique d'intérêt (immunogène) à un adjuvant. Bien que de nombreux types d'adjuvants soient en développement, les hydroxydes d'aluminium (alum) étaient jusqu'à récemment le seul adjuvant autorisé pour une administration à l'homme. Or l'alum est un adjuvant faible pour l'induction des réponses humorales, il n'est pas approprié pour tous les antigènes et ne permet pas d'obtenir des réponses cellulaires. Parmi les adjuvants en développement, les systèmes les plus efficaces pour induire une immunité forte sont les vecteurs synthétiques à base de microparticules et en particulier les microparticules de poly(lactide-co-glycolide) (PLGA) et de poly(acide lactique) (PLA). De par leur taille, ces particules permettent de cibler des cellules présentatrices d'antigène comme les macrophages ou les cellules dendritiques et améliorent la prise en charge de l'immunogène par le système immunitaire. La substance protéique peut être soit encapsulée dans les microparticules, soit adsorbée à la surface. Les deux approches permettent d'induire à la fois des réponses immunes cellulaires et humorales, même si à l'origine elles étaient surtout utilisées pour obtenir une réponse anticorps. Cependant, l'encapsulation présente deux inconvénients majeurs. Le premier c'est qu'il faut une dégradation partielle ou totale de la microparticule pour que la substance protéique devienne disponible pour le système immunitaire, ainsi la mise en place de l'immunité est retardée. Le second c'est que la substance protéique peut être dénaturée ou dégradée lors de l'encapsulation et ainsi perdre ses propriétés immunogéniques. Pour ces raisons ce procédé ne peut pas être utilisé avec tout type de substance protéique, alors que le procédé par adsorption ne présente pas ces inconvénients. Dans les deux cas, l'obstacle majeur à l'utilisation est leur manque d'efficacité pour générer des réponses cellulaires spécifiques. Si par rapport à l'alum les réponses cellulaires induites sont bien meilleures, elles sont bien en deçà de ce que l'on peut obtenir avec un vecteur viral.

Les vecteurs viraux recombinants permettent de faire exprimer in vivo (chez l'animal ou chez l'homme) la substance protéique d'intétêt. Il s'agit d'une stratégie efficace qui permet une forte mobilisation du système immunitaire. Mais l'induction d'une réponse immune durable dans le temps nécessite l'utilisation de vecteurs vivants atténués qui peuvent se répliquer dans l'organisme vacciné. Or, pour des raisons réglementaires liées à la sécurité d'utilisation des vaccins, les nouvelles générations de vaccins utilisent des vecteurs viraux non réplicatifs chez l'homme, entraînant une diminution des réponses immunes spécifiques dans le temps. Ainsi, dans le cas de vaccins prophylactiques, il est encore parfois nécessaire de restimuler l'immunité spécifique pour garder une réponse mémoire efficace. Dans le domaine de l'immunothérapie, la restimulation de l'immunité spécifique reste utile pour maintenir l'intensité des réponses immunes qui permettent de contrôler l'organisme pathogène. Ainsi, l'administration répétée de vecteurs viraux semble nécessaire mais s'accompagne d'un problème majeur qui est celui de l'immunité anti- vecteur. En effet, le vecteur viral est lui-même un immunogène et dès lors qu'il est administré à plusieurs reprises, il y a apparition puis augmentation de l'immunité spécifique du vecteur. Cette réponse immune visant à éliminer le vecteur viral empêche aussi l'expression du gène qu'il véhicule entraînant ainsi une diminution de l'efficacité des réponses immunes contre la substance protéique d'intérêt. De plus, l'immunité anti- vecteur empêche l'utilisation du vecteur concerné pour vacciner contre une autre maladie. Schagen et al. décrivent certaines approches permettant de pallier cette immunité anti- vecteur telles que des modifications au niveau du vecteur ou encore des adaptations au niveau des patients vaccinés (2004, Critical reviews in Oncology/Hematology 50 :51-70). Toutefois la mise en oeuvre de ces approches est difficile et ne peut pas être réalisée pour toutes les maladies ou tous les patients. La stratégie utilisant des vecteurs viraux n'est donc pas optimisée.

Une autre voie d'amélioration de la réponse immune induite par vaccination est la stratégie de prime-boost. Elle consiste à augmenter les réponses immunes spécifiques en administrant séquentiellement deux vaccins différents véhiculant une même substance protéique au lieu d'administrer le même vaccin plusieurs fois. Par exemple, il est possible d'utiliser un vecteur viral différent à chaque injection (Negri DR, et al., 2004, Journal of Général Virology, 85 :1191-1201). Cependant cette stratégie n'a que peu de chances de succès car le nombre de vecteurs viraux dont l'emploi est autorisé chez l'homme est très restreint. De plus, l'administration répétée de ces vecteurs viraux se heurte encore une fois au problème de l'immunité anti-vecteur. Afin de contourner ce problème, Otten et al. décrivent l'utilisation d'un prime ADN suivi d'un boost de microparticules de PLGA en surface desquelles des antigènes protéiques sont adsorbés (2003, Journal of Virology, 77 :6087-6092). Sharpe et al. ont proposé un prime ADN en encapsulant l'ADN dans des microparticules à base de PLGA et un boost par un vecteur viral MVA (2003, Virology, 313 :13-21). O'hagan et aL décrivent l'utilisation d'un prime par de l'ADN adsorbé sur des microparticules de PLGA, suivi d'un boost par un vecteur vaccine (2001, Journal of Virology, 75 :9037-9043). La demande de brevet WO 98/56919A décrit un prime ADN, suivi d'un boost par un poxvirus. L'inconvénient de l'utilisation d'un prime ADN est sa faible efficacité notamment chez les primates et aussi lorsqu'il s'agit de gène peu immunogène comme par exemple le gène *tat* du VIH-1. La demande de brevet WO 98/56919A décrit également l'utilisation d'un prime par des VLP (« virus-like particles »), suivi d'un boost par un poxvirus. Les VLP sont des particules virales défectives qui n'incorporent pas le génome viral et qui ne peuvent pas se répliquer. Elles sont souvent formées par autoassemblage des protéines structurales d'un virus ou d'un rétrotransposon (élément endogène semblable à un rétrovirus) telles que le précurseur Pr55 Gag du HIV-1 ou les protéines L1 et L2 du HPV-16 (papillomavirus humain). En fonction des protéines virales exprimées, la structure du VLP peut se rapprocher plus ou moins de celle du virion natif, de sorte que les VPL sont comparables à un virus. L'inconvénient de ces VLP est qu'elles sont issues de la biotechnologie et que leur fabrication selon les normes réglementaires est très difficile.

Les Demanderesses ont maintenant mis en évidence, contre tout attente, que l'association particulière d'un vecteur synthétique colloïdal biorésorbable comprenant des antigènes protéiques et d'un vecteur viral comprenant les séquences nucléotidiques codant pour les antigènes protéiques correspondantes, permet d'augmenter l'efficacité de la vaccination liée à l'utilisation d'une antigène protéique, tout en diminuant l'immunité anti-vecteur.

Ainsi un premier objet de l'invention consiste en une composition pharmaceutique comprenant ou consistant en :
- un vecteur synthétique colloïdal constitué de microparticules biorésorbables non toxiques comprenant une antigène protéique ou plusieurs antigènes protéiques, et
- un vecteur viral comprenant au moins une séquence nucléotidique codant pour une ou des antigènes protéique(s) compris dans le vecteur synthétique
- éventuellement en association avec un excipient pharmaceutiquement acceptable.

Un autre objet de l'invention consiste en l'utilisation de ladite composition pour la préparation d'un médicament, de préférence un vaccin. Elle peut également être utilisée pour le diagnostic *in vitro* d'états pathologiques relatifs aux substances protéiques de la composition de l'invention.

La présente invention propose donc une nouvelle composition comprenant un vecteur synthétique colloïdal constitué de microparticules biorésorbables présentant au moins un antigène protéique.

On entend par composé biorésorbable un composé qui n'est pas toxique, c'est-à-dire dont l'administration dans l'organisme ne provoque pas de modification physiologique ou métabolique létale, invalidante ou gênante dans la vie quotidienne, et qui peut être éliminé par les voies naturelles de l'organisme après administration.

Les termes colloïdes et colloïdal(aux) définissent toute dispersion d'une substance (phase dispersée) au sein d'une phase continue aqueuse dont au moins une des trois dimension est inférieure ou égale à 10µm et préférentiellement inférieure ou égale à 1µm.

Une phase continue aqueuse est une phase dont la composition est majoritairement de l'eau, comme par exemple un tampon ou un mélange eau/solvant organique miscible à l'eau. Bien entendu, une phase continue aqueuse appropriée aux fins de l'invention est une phase non toxique.

La phase dispersée peut être un liquide insoluble dans la phase continue comme par exemple une huile ou un mélange d'huiles, ou bien des matières grasses organiques, ou encore être un solide de nature polymère ou minérale. La phase dispersée est toujours biorésorbable.

Des exemples de phase dispersée comprennent l'huile d'olive, l'huile de tournesol, l'huile d'arachide, l'huile de colza, ou leurs mélanges ; des triglycérides ; les polymères synthétiques tels que les polymères d'hydroxyacide comme par exemple l'acide lactique, l'acide glycolique, l'aide hydroxybutyroique, ou les composés issus de la caprolactone ; des polypeptides comme les composés issus de la polymérisation du caprolactame ; les polymères naturels tels que, à titre d'exemple, les polysaccharides comme l'acide hyaluronique, le chitosane, le dextrane.

Des exemples de tels colloïdes biorésorbables sont donnés dans Benita S. et Levy M.Y., 1993, Journal of Pharmaceutical Sciences, 82(11) :1069-1079 (émulsions liquide/liquide), Trotta M., et al., 2003, International Journal of Pharmeaceutics, 257 : 153-160 (émulsions lipide solide/liquide) et Jiang W., et al., 2005, Biodegradable poly(lactic-co-glycolic) acid microparticles for injectable delivery of vaccine antigens Advanced Drug Delivery Reviews, 57 : 391-410.

La forme du vecteur synthétique colloïdal est donnée par la phase dispersée qui peut être sphérique ou non, homogène ou constituée d'un empilement de lamelles.

De préférence, le vecteur synthétique de l'invention est de nature sphérique. De préférence encore le vecteur est composé de microsphères. Les termes microsphère et microparticule sont employés indifféremment dans la suite de la description. Par microparticule ou microsphère on entend des particules de taille au plus micronique pour leur permettre d'entrer dans les cellules présentatrices d'antigènes. Par taille au plus micronique, on entend une dimension inférieure ou égale à 999 µm. De préférence, les microparticules ont un diamètre de particule inférieure ou égale à 3 µm. De façon davantage préférée, les particules de l'invention sont de taille submicronique, avec, de préférence, un diamètre compris entre 150 et 900 nm, de préférence encore entre 250 et 700 nm. La taille des particules est facilement déterminée par des techniques connues de l'homme du métier telles que, par exemple, en utilisant la microscopie à balayage électronique, la diffusion quasi-élastique de la lumière, la microscopie électronique à transmission.

Selon un mode de réalisation de l'invention, les vecteurs synthétiques colloïdaux sont des vecteurs à base de polymère.

A titre d'exemple de vecteurs synthétique à base de polymère, on peut citer des vecteurs comprenant des microparticules à base de polymères tels que les poly(acides α-hydroxylés), les poly(acides hydroxybutyriques), les polycaprolactonés, les polyorthoesters et les polyanhydrides. De préférence, le vecteur synthétique comprend au moins un polymère biorésorbable choisi parmi un poly(acide α-hydroxylé) tel que le poly(acide D-lactique), le poly(acide L-lactique) (appelés PLA), le poly(acide glycolique) (appelé PLG), ou bien un mélange de poly(acides α-hydroxylés) tels qu'un mélange de poly(acides D- et L-lactiques), un mélange de poly(acide L-lactique) et de poly(acide glycolique), un mélange de poly(acide D-lactique) et de poly(acide glycolique), ou un mélange de poly(acides D- et Lactiques) et de poly(acide glycolique). Lorsque le polymère est un mélange de poly(acides α-hydroxylés), la proportion de chaque constituant peut être facilement déterminée par l'homme du métier. Ainsi, par exemple, on peut utiliser un mélange racémique de poly(acides D- et L-lactiques) ou un mélange PLA/PLG à divers pourcentages connus de l'homme du métier. De préférence, le vecteur synthétique colloïdal selon l'invention est préparé à partir d'au moins un polymère choisi parmi les poly(acides α-hydroxylés). De préférence encore, les particules de l'invention sont dénuées d'agent stabilisant et d'agent tensioactif de sorte qu'elles ne sont pas toxiques. Par microparticule toxique, on entend une microparticule comportant au moins un composé susceptible de provoquer des troubles biologiques, tels que des perturbations du métabolisme, dans l'organisme ayant reçu la microparticule. De telles particules non toxiques peuvent être préparées selon le protocole décrit dans la demande de brevet W02005/027871.

Selon un autre mode de réalisation, le vecteur synthétique colloïdal est préparé à partir d'au moins un polymère naturel tel que ceux cités précédemment.

La composition de l'invention est utile pour stimuler tant une réponse à médiation cellulaire qu'une réponse humorale. Elle est utile tant en prophylactique et thérapeutique qu'en diagnostic.

Ainsi, les substances protéiques appropriées aux fins de l'invention portées par le vecteur synthétique peuvent être toutes substances protéiques contre lesquelles on cherche à déclencher une réponse immunitaire.

Par réponse immunitaire, on entend une réponse à médiation cellulaire, une réponse humorale ou les deux.

Par réponse à médiation cellulaire, on entend une réponse médiée par les lymphocytes T et/ou d'autres leucocytes. Cette réponse se traduit par l'induction d'une activité lytique par les lymphocytes T cytotoxiques et/ou par la production de cytokines par les lymphocytes T CD8+ ou par les cellules T auxiliaires (helper).

Par réponse humorale, on entend une réponse médiée par les molécules anticorps sécrétés par les lymphocytes B.

Par substance protéique, on entend toute substance protéique ayant un rôle d'antigène après addition aux vecteurs appropriés aux fins de l'invention tels que peptides, protéines, fragments de protéine, polyprotéines, glycoprotéines et glycoprotéines, étant entendu que les fragments de protéine ont conservé la structure d'intérêt.

Les antigènes sont des molécules susceptibles d'être reconnues par un anticorps dont ils ont induit la synthèse par une réponse immunitaire et contenant au moins un épitope.

Des exemples de fragments de protéine comprennent par exemple les peptides, les polypeptides et les épitopes.

Les épitopes sont des peptides comprenant entre 3 et 15 et généralement entre 5 et 15 acides aminés, consistant en le fragment peptidique minimal contre lequel est déclanché une réponse immunitaire.

Le vecteur synthétique présente les substances protéiques en de multiples copies. Par au moins une substance protéique, on entend au moins plusieurs substances protéiques de même type. Par type de substance protéique, on entend un ensemble de substances protéiques déclanchant la même réponse immunitaire par production des mêmes anticorps (i.e. ayant la même immunité).

Le vecteur synthétique peut donc comprendre :
- soit des substances protéiques de même type et donc constituant des antigènes associé à la même maladie. Ainsi, par exemple, le vecteur synthétique peut comprendre comme substance protéique la protéine p24 sous forme de protéine native et/ou de protéine mutée et/ou de fragment de protéine, étant entendu que la protéine mutée et le fragment ont conservé leur immunité ;
- soit des substances protéiques de types différents et constituant des antigènes associé à la même maladie. Ainsi, par exemple, le vecteur synthétique peut comprendre plusieurs protéines du virus de l'immunodéficience humaine (VIH) telles que la protéine Gag, la protéine Gag mutée n'ayant pas la même immunité, la protéine Tat, la protéine Rev, la protéine Nef, la protéine Env ;
- soit des substances protéiques de types différents et constituant des antigènes associé à des maladies différentes. Ainsi, par exemple, le vecteur synthétique peut comprendre au moins un type de protéine du VIH, telles que les protéines p24, Tat, Rev, et au moins un type de protéine du virus de l'hépatite C (VHC) telles que les protéines NS2, NS3, NS4.

Selon un mode de réalisation de l'invention, le vecteur synthétique comprend des substances protéiques de types différents et constituant des antigènes associés à la même maladie.

Les substances protéiques peuvent être de plusieurs origines telles que d'origine virale, bactérienne ou de pathologie d'origine non infectieuse (comme le cancer ou une maladie métabolique).

Selon un mode de réalisation particulier de l'invention, la substance protéique est un antigène d'origine virale.

Lorsque la substance protéique est d'origine virale, les virus utilisés sont tous virus pour lesquels on connaît des substances capables d'une réponse immunitaire.

A titre d'exemple, on peut citer, sans aucune limitation, les herpes virus, les virus des hépatites tels que le virus de l'hépatite B (VHB) ou le virus de l'hépatite C (VHC), les papillomas virus (HPV),les virus de l'immunodéficience humaine (VIH), tels que VIH-1 et VIH-2, différentes souches des virus de grippe humaine et de grippe aviaire.

Les séquences nucléiques des virus appropriées aux fins de l'invention, ainsi que les protéines codées par lesdites séquences sont largement connues de l'homme du métier et sont disponibles par exemple dans des bases de données telles que GenBank.

Ainsi, par exemple, le virus VIH a des gènes qui codent pour des protéines structurelles du virus. Le gène *gag* code pour la protéine qui forme le core du virion, incluant l'antigène p24. Le gène *pol* code pour les enzymes responsables de la transcription inverse (transcriptase inverse), du clivage (protéase) et de l'intégration (intégrase). Le gène *env* code pour les glycoprotéines d'enveloppe. II contient six autres gènes (*tat, rev, nef, vif, vpr* et *vpu* (VIH-1) ou *vpx* (VIH-2)) qui codent pour des protéines impliquées dans la régulation de l'expression des gènes du virus (protéines de régulation). Le génome du VIH comprend également les LTR 5' et 3' (Long Terminal Repeat) qui comprennent des éléments de régulation impliqués dans l'expression des gènes du virus.

Selon un mode de réalisation de l'invention, la substance protéique utilisée dans le procédé de l'invention est une protéine du virus VIH. A titre d'exemple d'antigène du virus VIH, on peut citer les protéines de régulation ou la protéine gag ou la glycoprotéine Env, les protéines de régulation préférées étant la protéine Tat, Rev ou Nef.

S'agissant du VHC, l'extrémité 5' de son génome correspond à une région non traduite adjacente aux gènes qui codent pour les protéines structurales, la protéine core de la nucléocapside, les deux glycoprotéines d'enveloppe, E1 et E2, et une petite protéine appelée p7. La région non traduite 5' et le gène core sont relativement bien conservés dans les différents génotypes. Les protéines d'enveloppe E1 et E2 sont codées par des régions plus variables d'un isolat à un autre. La protéine p7 est une protéine extrêmement hydrophobe qui constituerait un canal ionique. L'extrémité 3' du génome du VHC contient les gènes qui codent pour les protéines non structurales (NS2, NS3, NS4, NS5) et pour une région 3' non codante possédant un domaine bien conservé (Major ME, Feinstone SM, Hepatology, juin 1997, 25(6) : 1527-1538).

La protéine non structurale NS3 du VHC est une protéine de 630 acides aminés qui comprend deux domaines structuraux distincts : un domaine N-terminal, de 81 acides aminés, doté d'une activité protéasique à sérine active intervenant dans la maturation de la protéine virale (domaine appelé NS3 protéase), et un domaine C-terminal, de 549 acides aminés, comprenant une activité hélicase associée à une activité NTPasique qui joue un rôle dans la réplication du génome viral (domaine appelé NS3 hélicase). Cette protéine NS3 est relativement bien conservée parmi les différents génotypes du virus, de sorte que cette protéine constitue un antigène « candidat vaccin » de choix.

Selon un autre mode de réalisation de l'invention, la substance protéique est un antigène associé aux tumeurs.

En effet, certaines tumeurs expriment des antigènes qui sont potentiellement reconnus par les lymphocytes T CD8+ même s'ils ne sont pas à l'origine d'une réponse immune intense. Il s'agit d'antigènes associés aux tumeurs (AAT) ou marqueurs tumoraux (Houghton AN, 1994, J Exp Med 180:1). En améliorant la présentation des AAT au système immunitaire, il est possible de les rendre plus immunogéniques, ainsi les AAT peuvent servir de base au développement de vaccins anti-cancéreux sous forme de protéine recombinante ou de peptide de synthèse.

Afin de tester les nouvelles stratégies d'immunothérapie qui visent à augmenter la reconnaissance des AAT, Wang et al. ont développé un modèle expérimental chez la souris (1995, Journal of Immunology, 154:4685-4692). La lignée tumorale CT26.WT, fond génétique BALB/c, a été transduite par le gène lacZ d'*Escherichia coli* codant pour l'enzyme beta-galactosidase afin de créer la lignée CT26.CL25. Les deux lignées sont létales chez la souris et leur cinétique de croissance sont similaires. Administrée chez la souris, la lignée CT26.CL25 est à l'origine de formation de tumeurs qui peuvent être ralenties voire inhibées en présence d'une réponse immune contre la beta-galactosidase qui constitue un AAT modèle.

Les substances protéiques appropriées aux fins de l'invention peuvent être obtenues par les techniques du génie génétique qui comprend les étapes de :
- culture d'un microorganisme ou de cellules eucaryotes transformé(es) à l'aide d'une séquence nucléotidique codant pour la substance protéique d'intérêt et
- récupération de ladite substance protéique produite par ledit microorganisme ou lesdites cellules eucaryotes.

Ces techniques sont bien connues de l'homme du métier. Pour plus de détail la concernant, on pourra se référer à l'ouvrage ci-après : Recombinant DNA Technology I, Editors Ales Prokop, Raskesh K Bajpai ; Annals of the New-York Academy of Sciences, Volume 646, 1991.

Les substances protéiques d'intérêt, lorsqu'elles sont de petites tailles, peuvent également être préparées par les synthèses peptidiques classiques bien connues de l'homme du métier.

La substance protéique est associée aux vecteurs colloïdaux selon les techniques connues de l'homme de l'art, telles que l'inclusion ou la liaison à la surface. A titre d'exemple d'inclusion de la substance protéique dans le vecteur synthétique colloïdal, on peut citer l'encapsulation (Tamber H, et al., 2005, Adv Drug Deliv Rev, 57(3): 357-76.). A titre de liaison de la substance colloïdale au vecteur synthétique colloïdal, on peut citer l'adsorption, comme décrit dans la demande de brevet WO2005/027871, ou le greffage covalent impliquant la formation d'une liaison chimique entre un groupement réactif du vecteur colloïdal et un autre de la substance protéique de façon décrite par exemple dans 'Protein immobilizadon-Fundamentals and Applications R.F. Taylor Ed, Marcel Dekker inc, New York, 1991.

Selon un mode de réalisation particulier, les substances protéiques sont ajoutées aux vecteurs synthétiques par liaison et de préférence encore par adsorption, comme par exemple en mélangeant les microparticules avec les substances protéiques et en incubant sous agitation, par exemple à température ambiante ou à 37°C.

La composition selon l'invention comprend également un vecteur viral.

Le vecteur viral comprend au moins une séquence nucléotidique codant pour une substance protéique correspondant à au moins une substance protéique du vecteur synthétique.

Par substance protéique du vecteur viral correspondant à une substance protéique du vecteur synthétique, on entend que la substance protéique du vecteur viral constitue un antigène associé à la même maladie que la substance protéique du vecteur synthétique, qu'elle soit du même type ou de type différent, comme indiqué précédemment. Quand les substances protéiques sont de type différent, il faut qu'elles aient au moins un épitope en commun, comme par exemple les protéines Gag et p24.

Ainsi, le génome du vecteur viral est modifié de façon à insérer une ou plusieurs séquences nucléotidiques codant pour une ou plusieurs substances protéiques constituant des antigènes de la même maladie ou des maladies différentes. Parmi ces substances protéiques, au moins une correspond à une substance protéique du vecteur synthétique, ces substances protéiques constituant des antigènes associés à la même maladie.

Selon un mode de réalisation préféré, le vecteur viral comprend une séquence nucléotidique codant pour une protéine du VIH.

Outre la ou les séquences nucléotidiques codant pour au moins une substance protéique constituant un antigène associé à la même maladie que la substance protéique du vecteur synthétique, le vecteur viral comprend également les moyens nécessaires à l'expression de ladite substance protéique.

On entend par moyen nécessaire à l'expression d'une substance protéique, tout moyen qui permet d'obtenir la substance protéique, tel que notamment un promoteur, un terminateur de transcription, une origine de réplication et de préférence un marqueur de sélection.

Les moyens nécessaires à l'expression d'une substance protéique sont liés de façon opérationnelle à la séquence d'acide nucléique codant pour ladite substance. Par «liés de façon opérationnelle », on entend une juxtaposition desdits éléments nécessaires à l'expression et du gène codant pour la substance protéique, lesquels sont en une relation telle que cela leur permet de fonctionner de façon attendue. Par exemple, ils peut exister des bases supplémentaires entre le promoteur et le gène d'intérêt tant que leur relation fonctionnelle est préservée.

Les moyens nécessaires à l'expression de la substance protéique peuvent être des moyens homologues, c'est-à-dire inclus dans le génome du vecteur utilisé, ou bien être hétérologues. Dans ce dernier cas, lesdits moyens sont clonés avec la protéine d'intérêt à exprimer.

Des exemples de promoteurs hétérologues comprennent (i) les promoteurs viraux tels que le promoteur SV40 (Virus simien 40), le promoteur du gène de la thimidine-kinase du virus simplex de l'Herpès (TK-HSV-1), le LTR du virus du sarcome de Rous (RSV), le promoteur intermédiaire précoce du cytomégolovirus humain (CMV) et le promoteur majeur tardif adénoviral (MLP), ainsi que (ii) tout promoteur cellulaire qui contrôle la transcription des gènes codant pour des protéines chez des eucaryotes supérieurs, tel que le promoteur du gène de phosphoglycérate-kinase (PGK) constitutif (Adra et al., 1987, Gene, 60 : 65-74), le promoteur des gènes spécifiques du foie alphal-antitrypsine et FIX et le promoteur SM22 spécifique des cellules du muscle lisse (Moessler et al., 1996, Development, 122: 2415-2425).

Lesdites séquences contenues dans le vecteur viral peuvent être liées directement entre elles sous le contrôle d'un seul promoteur et/ou d'un seul élément régulateur de l'expression, ou bien elles peuvent être séparées en étant sous la dépendance chacune de promoteurs et/ou régulateurs de l'expression indépendants, identiques ou différents.

A titre de vecteur viral qui conviennent aux fins de l'invention, on peut citer par exemple les vecteurs viraux type adénovirus, poxvirus, baculovirus, herpesvirus, togavirus, rétrovirus.

Les adénovirus ont été détectés dans de nombreuses espèces animales. Ils ne s'intègrent pas et sont peu pathogènes. Ils sont capables d'infecter une variété de types cellulaires, les cellules en division et les cellules en repos. Ils possèdent un tropisme naturel pour les épithéliums bronchiques. De plus, ils ont été utilisés en tant que vaccins entériques vivants pendant de nombreuses années avec un excellent profile de sécurité. Enfin, on peut les faire pousser facilement et les purifiées en grande quantité. Ces caractéristiques ont fait que les adénovirus sont particulièrement appropriés pour une utilisation en tant que vecteurs d'expression et notamment en tant vecteurs de thérapie génique à des fins thérapeutiques et vaccinales.

Selon un mode de réalisation préféré, le vecteur de l'invention est un adénovirus.

Des exemples d'adénovirus à utiliser dans la présente invention peuvent être dérivés de toute source d'origine humaine ou animale, en particulier d'origine canine (par exemple CAV-1 ou CAV-2 ; référence Genbank CAV1GENOM et CAV77082, respectivement), d'origina avienne (référencé Genbank AAVEDSDNA), d'origine bovine (telle que BAV3, Seshidhar Reddy et al., 1998, J. Virol., 72 : 1394-1402), d'origine ovine, féline, porcine, d'origine simienne, ou bien d'un de leurs hybrides. Tout sérotype peut être utilisé. Toutefois, les adénovirus d'origine humaine sont préférés et en particulier l'adénovirus 5 (Ad5).

De façon générale, les virus cités sont disponibles dans les collections ATCC et ont fait l'objet de nombreuses publications décrivant leur séquence, leur organisation et leur biologie, ce qui permet à l'homme du métier de les appliquer facilement. Par exemple, la séquence de l'adénovirus type 5 est décrite dans la base de donnée Genbank (M73260 et M29978) et est incorporée ici par référence.

Le génome des adénovirus est constitué d'une molécule d'ADN linéaire double brin d'environ 36 kb portant plus d'environ 30 gènes nécessaires pour terminer le cycle viral. Il y a 4 gènes précoces répartis dans le génome de l'adénovirus (E1 à E4). Les régions E1, E2 et E4 sont essentielles pour la réplication virale. La région E3 est considérée comme une région non essentielle sur la base de l'observation que les virus mutants apparaissant naturellement ou les virus hybrides ayant perdu cette région E3 continuent à se répliquer comme les virus de type sauvage dans les cellules cultivées (Kelly et Lewis, 1973, J. Virol., 12 :643-652). Les gènes tardifs (L1 à L5) codent en majorité pour les protéines structurales constituant la capside virale. Ils chevauchent au moins en partie les premiers motifs de transcription et sont transcrits à partir d'un promoteur unique (MLP pour « Major Late Promoter »). De plus, le génome adénoviral porte aux deux extrémités des régions à action en cis essentielles pour la réplication d'ADN, respectivement les motifs de répétition inversés 5' et 3' (ITRs pour « Inverted Terminal Repeats ») et une séquence d'empaquetage.

Les adénovirus actuellement utilisés dans les protocoles de thérapie génique sont dénués de la majorité de la région E1, ce qui rend les virus déficients au niveau de leur réplication pour éviter leur dissémination dans l'environnement et dans l'organisme hôte. En outre, la plupart des adénovirus sont également dénués de la région E3 afin d'accroître leur capacité de clonage. La faisabilité du transfert de gène en utilisant ces vecteurs a été démontrée dans une variété de tissus *in vivo* (voir par exemple Yei et al., 1994, Hum. Gene Ther., 5 : 731-744 ; Dai et al., 1995, Proc. Natl. Acad Sci. USA, 92: 1401-1405 ; US6,099,831 ; et US6,013,638).

Un autre vecteur d'expression particulièrement approprié aux fins de l'invention est un poxvirus, lequel constitue un autre mode de réalisation de l'invention.

Les poxvirus constituent un groupe de virus complexe enveloppés, se distinguant principalement par leur morphologie inhabituelle, leur grand génome d'ADN et leur site cytoplasmique de réplication. Le génome de plusieurs éléments des *poxviridae,* comprenant la souche virale de la vaccine de Copenhagen (VV) (Goebel et al., 1990, Virol. 179 : 247-266 et 517-563) et la souche du virus de la vaccine modifié d'Ankara (MVA) (Antoine et al., 1998, Virol., 244 : 635-396), a été cartographié et séquencé. La souche VV possède un génome d'ADN double brin d'environ 192 kb codant pour environ 200 protéines dont approximativement 100 sont impliquées dans l'assemblage du virus. La souche MVA est une souche du virus de la vaccine hautement atténuée, générée par plus de 500 passages en série de la souche d'Ankara du virus de la vaccine (CVA) sur des fibroblastes d'embryons de poulet (Mayr et al., 1975, Infection, 3 : 6-16). Le virus MVA a été déposé devant la Collection Nationale de Cultures de Microorganismes (CNCM) sous le numéro 1-721. La détermination de la séquence complète du génome du MVA et la comparaison avec celui du VV permet l'identification précise des altérations qui sont apparues dans le génome viral et la définition de sept délétions (I à VII) et de nombreuses mutations conduisant à des cadres de lecture ouverts fragmentés (Antoine et al., 1998, Virology, 244 : 365-396).

D'autres exemples de poxvirus appropriés aux fins de l'invention comprennent le pox du canari, le pox de volaille, le pox de vache, l'entomopox, le pox de singe, le pox de porc et le pox de pingouin.

Le poxvirus se trouve sous deux formes morphologiquement distinctes, appelées virus mature intracellulaire (IMV) et virus extracellulaire enveloppé (EEV).

De préférence, le poxvirus utilisé aux fins de l'invention est un Modified Vaccinia Virus Ankara.

Les méthodes de suppression et d'insertion de séquences d'ADN dans des vecteurs d'expression sont largement connues de l'homme du métier et consistent notamment en des étapes de digestion enzymatique et ligature. De préférence la séquence nucléotidique insérée dans le vecteur viral code pour une protéine du virus du VIH.

Les compositions à base de vecteurs selon l'invention sont particulièrement efficaces pour l'inhibition, la prévention et le traitement d'une maladie dont la substance protéique de la composition constitue un antigène, telle qu'une infection par un virus. Elles sont donc particulièrement utiles pour la préparation d'un médicament, et en particulier d'un vaccin, qu'il soit thérapeutique ou prophylactique.

Dans une approche de traitement prophylactique ou thérapeutique, en particulier vaccination, une dose efficace de la composition selon l'invention est administrée à un patient.

Les compositions pharmaceutiques de l'invention sont appropriées pour l'administration orale, sublinguale, sous-cutanée, intramusculaire, intraveineuse, topique, locale, intratrachéale, intranasale, transdermique, rectale, vaginale, intraoculaire, intra-auriculaire, ledit principe actif pouvant être administré sous forme unitaire d'administration.

Les formes unitaires d'administration peuvent être par exemple des comprimés, des gélules, des granules, des poudres, des solutions ou suspensions orales injectables, des timbres transdermiques (« patch »), des formes d'administration sublinguale, buccale, intratrachéale, intraoculaire, intranasale, inàra-auriculaire, par inhalation, des formes d'administration topique, telles que rectale ou vaginale, transdermique, sous-cutanée, intramusculaire ou intraveineuse, ou des implants. Pour l'administration topique, on peut envisager des crèmes, gels, pommades, lotions ou collyres.

Ces formes galéniques sont préparées selon les méthodes usuelles des domaines considérés.

Lesdites formes unitaires sont dosées pour permettre une administration journalière de 0,001 à 10 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse du patient.

La quantité et la nature de l'excipient peuvent être facilement déterminées par l'homme du métier. Elles sont choisies selon la forme pharmaceutique et le mode d'administration souhaités.

La composition selon l'invention peut être contenus dans un kit pharmaceutique. L'administration de la composition selon l'invention peut être simultanée, séparée ou échelonnée dans le temps.

Par administration simultanée, on entend une administration au même moment et au même endroit de la composition.

Par administration échelonnée, on entend l'administration du vecteur viral ou du vecteur synthétique à des temps différés. Le délai entre chaque administration peut s'étendre de quelques minutes à plusieurs années, de préférence de plusieurs semaines à plusieurs mois et de façon préférée elle peut être de 3 ou 4 semaines.

Par administration séparée, on entend l'administration du vecteur viral et du vecteur synthétique à des endroits corporels différents. Le vecteur viral et le vecteur synthétique peuvent être dans le même excipient ou de préférence dans des excipients différents appropriés.

De préférence, la composition selon l'invention est administrée de façon échelonnée. De préférence encore, le vecteur synthétique est administré avant le vecteur viral.

Ainsi, selon un mode de réalisation de l'invention, la composition de l'invention comprend :
i) un constituant prime consistant en le vecteur synthétique colloïdal biorésorbable comprenant au moins une substance protéique et
ii) un constituant boost consistant en le vecteur viral comprenant au moins une séquence nucléotidique codant pour une substance protéique correspondant à au moins une substance protéique du vecteur synthétique.

Outre une application thérapeutique et prophylactique, l'invention a également une application diagnostique. Ainsi, l'invention concerne également l'utilisation de la composition de l'invention pour le diagnostic *in vitro* de l'état pathologique dont la ou les substances protéiques du vecteur synthétique et du vecteur synthétique constituent les antigènes, et en particulier d'une infection virale ou un cancer.

La présente invention sera mieux comprise à l'aide des exemples suivants donnés uniquement à titre illustratif et non limitatif, ainsi qu'à l'aide des figures 1 à 20 annexées, sur lesquelles :
La figure 1 représente les réponses CTL lors de l'expérience d'immunisation N°1 avec différentes compositions vaccinales dont les compositions selon l'invention comprenant un vecteur synthétique à base de microparticules de poly(acidé D,L-lactique) associées à la protéine p24 (appelé ci-après PLA/p24) et d'un vecteur viral MVA comprenant la séquence gag (appelé ci-après virus MVA gag).
La figure 2 représente les réponses ELISPOT IFN-γ spécifiques du peptide Gag AMQ lors des 3 expériences d'immunisation avec différentes compositions vaccinales dont les compositions selon l'invention comprenant le vecteur synthétique PLA/p24 et le vecteur viral MVA gag.
La figure 3 représente les réponses ELISPOT IFN-γ spécifiques de la protéine p24 lors des 3 expériences d'immunisation avec différentes compositions vaccinales dont les compositions selon l'invention comprenant le vecteur synthétique PLA/p24 et le vecteur viral MVA gag.
La figure 4 représente les réponses ELISPOT IL-4 spécifiques de la protéine p24 lors des 3 expériences d'immunisation avec différentes compositions vaccinales dont les compositions selon l'invention comprenant le vecteur synthétique PLA/p24 et le vecteur viral MVA gag.
La figure 5 représente la sécrétion de la cytokine GM-CSF spécifique de la protéine p24 lors des 3 expériences d'immunisation avec différentes compositions vaccinales dont les compositions selon l'invention comprenant le vecteur synthétique PLA/p24 et le vecteur viral MVA gag.
La figure 6 représente la sécrétion de la cytokine IL-5 spécifique de la protéine p24 lors des 3 expériences d'immunisation avec différentes compositions vaccinales dont les compositions selon l'invention comprenant le vecteur synthétique PLA/p24 et le vecteur viral M VA gag.
La figure 7 représente la sécrétion de la cytokine IL-2 spécifique de la protéine p24 lors des 3 expériences d'immunisation avec différentes compositions vaccinales dont les compositions selon l'invention comprenant le vecteur synthétique PLA/p24 et le vecteur viral MVA gag.
La figure 8 représente la cinétique d'apparition des réponses humorales spécifiques de la protéine p24 lors des 3 expériences d'immunisation avec différentes compositions vaccinales dont les compositions selon l'invention comprenant le vecteur synthétique PLA/p24 et le vecteur viral MVA gag.
La figure 9 représente les réponses CTL lors de l'expérience d'immunisation N°2 avec différentes compositions vaccinales dont les compositions selon l'invention comprenant le vecteur synthétique PLA/p24 et le vecteur viral Adénovirus Gag.
La figure 10 représente les réponses ELISPOT IFN-γ spécifiques du peptide Gag AMQ après immunisation avec différentes compositions vaccinales dont les compostions selon l'invention comprenant le vecteur synthétique PLA/p24 et le vecteur viral Adénovirus Gag.
La figure 11 représente la sécrétion spécifique de la protéine p24 des cytokines IL-2, IL-4, IL-10 après immunisation avec différentes compositions vaccinales dont les compositions selon l'invention comprenant le vecteur synthétique PLA/p24 et le vecteur viral Adénovirus Gag.
La figure 12 représente la cinétique d'apparition des réponses humorales spécifiques de la protéine p24 (12A) ou de l'adénovirus (12B) après immunisation avec différentes compositions vaccinales dont les compositions selon l'invention comprenant le vecteur synthétique PLA/p24 et le vecteur viral Adénovirus Gag.
La figure 13 compare les réponses ELISPOT IFN-γ spécifiques du peptide Gag AMQ après immunisation avec différentes compositions vaccinales dont les compositions comprenant PLA/p24 et MVA gag ou comprenant Alum/p24 et MVA gag.
La figure 14 compare les réponses ELISPOT IL-4 spécifiques de la protéine p24 après immunisation avec différentes compositions vaccinales dont les compositions comprenant PLA/p24 et MVA gag ou comprenant Alum/p24 et MVA gag.
La figure 15 compare les cinétiques d'apparition des réponses humorales spécifiques de la protéine p24 après immunisation avec différentes compositions vaccinales dont les compositions comprenant PLA/p24 et MVA gag ou comprenant Alum/p24 et MVA gag.
La figure 16 compare les cinétiques d'apparition des réponses humorales spécifiques du virus MVA après immunisation avec différentes compositions vaccinales dont les compositions comprenant PLA/p24 et MVA gag ou comprenant Alum/p24 et MVA gag.
La figure 17 représente les réponses ELISPO IFN-γ spécifiques du peptide beta-gal TPH après immunisation de souris avec différentes compositions vaccinales dont les compositions de l'invention comprenant le vecteur synthétique PLA-beta-gal et le vecteur viral MVA beta-gal.
La figure 18 compare les cinétiques d'apparition des réponses humorales spécifiques de la beta-gal après immunisation de souris avec différentes compositions vaccinales dont les compositions de l'invention comprenant le vecteur synthétique PLA-beta-gal et le vecteur viral MVA beta-gal.
La figure 19 représente les réponses ELISPOT IFN-γ spécifiques du peptide p24 AMQ après immunisation de souris avec différentes compositions vaccinales dont les compositions de l'invention comprenant le vecteur synthétique CPL-p24 et le vecteur viral MVA gag.
La figure 20 compare les cinétiques d'apparition des réponses humorales spécifiques du peptide p24 AMQ après immunisation de souris avec différentes compositions vaccinales dont les compositions de l'invention comprenant le vecteur synthétique CPL-p24 et le vecteur viral MVA gag.

### Exemple 1 : Immunisation de souris avec une composition vaccinale selon l'invention

La composition vaccinale utilisée comprend un vecteur synthétique à base de microparticules de poly(acide D,L-lactique) associées à la protéine p24 (appelé ci-après PLA/p24) et d'un vecteur viral MVA comprenant la séquence gag de SEQ ID N°1 (appelé ci-après virus MVA gag)

### 1. Modèle animal

Les expériences d'immunisation ont été réalisées chez des souris BALB/c (H-2K^{d}) femelles âgées de 6 à 8 semaines au moment de la première immunisation.

### 2. Immunogènes administrés

Dans cette série d'expériences on a utilisé les immunogènes suivants seuls ou en combinaison :
- les microparticules PLA/p24 préparées selon le protocoles déplacement de solvant et d'adsorption de la demande de brevet W02005/027871.
- le virus MVA gag (Modified Vaccinia Virus Ankara, virus de la vaccine atténué, souche Ankara) qui est recombinant pour le gène gag du virus VIH-1, codant pour la protéine p24. Le virus recombinant a été construit à Transgene à partir de la souche sauvage (MVATGN33) telle que décrite dans Antoine G., et al., 1998,«The complete genomic sequence of the modified vaccinia Ankara strain : comparison with other orthopoxvimses», Virology, 244 : 365-396. Ce virus recombinant a ensuite été produit et purifié comme suit : le MVA est répliqué sur les cellules embryonnaires de poulet. Après 48 à 72 h d'infection, les cellules sont récoltées, puis lysées par congélation/décongélation et par sonication. Le lysat est purifié/concentré par 2 coussins de saccharose suivi éventuellement de 2 gradients de saccharose.
Pour des précisions, on pourra se rapporter au livre « Current Protocols in Molecular Biology », Vol 2, chapitre 16.16 éditeurs Ausubel FM, Brent R, Kigston RE, Moore DD, Seidman JG, Smith JA, Struhl K, John Wiley & Sons, inc, New York. 3 volumes.
- le virus MVA sauvage MVATGN33. Il s'agit d'un témoin négatif qui sert à mesurer si le vecteur viral induit une réponse immune non spécifique lors des immunisations.
- l'ADN gag. Le gène gag (de SEQ ID N°1) a été cloné dans le plasmide d'expression eucaryote phCMV (contient le promoteur intermédiaire/précoce du cytomégalovirus humain et des signaux d'épissage et de polyadénylation issus du gène beta-globine du lapin (J K Yee, et al, 1994, Proc Natl Acad Sci U S A., 91(20): 9564-9568). Les préparations d'ADN sans endotoxines et en grande quantité ont été réalisées avec le kit de Gigaprep endotoxine-free de Macherey-Nagel. L'ADN sert de contrôle positif, car il est connu que chez la souris l'administration par voie intramusculaire d'ADN nu permet d'induire une bonne réponse cytotoxique (CTL).

### 3. Immunisations

### Expérience 1

35 souris ont reçu 2 doses des immunogènes décrits dans le point 2 ci-dessus à 0 et 3 semaines. Les compositions vaccinales reçues par chaque groupe de souris sont indiquées dans le tableau 1 ci-dessous. Toutes les injections ont été réalisées par voie sous-cutanée sauf pour l'ADN qui a été administré par voie intramusculaire.

**Tableau 1**

| Dose | Dose | Nombre de souris |
|---|---|---|
| 1^{ère} injection | 2^{ème} injection | |
| semaine 0 | semaine 3 | |
| MVA gag 10⁷ pfu | MVA gag 10⁷ pfu | 5 |
| MVA gag 10⁷ pfu | PLA/p24 40 µg | 5 |
| PLA/p24 40 µg | MVA gag 10⁷ pfu | 5 |
| PLA/p24 40 µg | PLA/p24 40 µg | 5 |
| MVA sauvage 10⁷ pfu | MVA sauvage 10⁷ pfu | 5 |
| ADN gag 10 µg (IM) | PLA/p24 40 µg | 5 |
| PLA/p24 40 µg | ADN gag 10 µg (IM) | 5 |

Pfu = plaque forming units, c'est l'unité de mesure de la dose de MVA utilisée. Le titre viral est exprimé en pfu selon la technique de dosage du stock viral utilisée, comme indiqué dans Current Protocols in Molecular Biology, *supra.*

Les animaux ont été sacrifiés 6 semaines après la première injection et le sang et la rate ont été prélevés pour les analyses immunologiques.

### Expérience 2

Dans cette expérience, on a augmenté le nombre d'injections.
30 souris ont reçu 2 ou 3 doses des immunogènes décrits dans le point 2 ci-dessus à 0, 3 et 6 semaines. Les compositions vaccinales reçues par chaque groupe de souris sont indiquées dans le tableau 2 ci-dessous. Toutes les injections ont été réalisées par voie sous-cutanée.

**Tableau 2**

| Dose | Dose | Dose | Nombre de souris |
|---|---|---|---|
| 1^{ère} injection | 2^{ème} injection | 3^{ème} injection | |
| Semaine 0 | Semaine 3 | Semaine 6 | |
| MVA gag 10⁷ pfu | MVA gag 10⁷ pfu | | 5 |
| MVA gag 10⁷ pfu | MVA gag 10⁷ pfu | MVA gag 10⁷ pfu | 5 |
| MVA gag 10⁷ pfu | PLA/p24 40 µg | | 5 |
| MVA gag 10⁷ pfu | PLA/p24 40 µg | PLA/p24 40 µg | 5 |
| PLA/p24 40 µg | MVA gag 10⁷ pfu | | 5 |
| MVA sauvage 10⁷ pfu | MVA sauvage 10⁷ pfu | MVA sauvage 10⁷ pfu | 5 |

Les animaux ont été sacrifiés 3 semaines après la dernière injection et le sang et la rate ont été prélevés pour les analyses immunologiques.

### Expérience 3

30 souris ont reçu 2 ou 3 doses des immunogènes décrits dans le point 2 ci-dessus à 0, 3 et 6 semaines. Les compositions vaccinales reçues par chaque groupe de souris sont indiquées dans le tableau 3 ci-dessous. Toutes les injections ont été réalisées par voie sous-cutanée.

**Tableau 3**

| Dose | Dose | Dose | Nombre de souris |
|---|---|---|---|
| 1^{ère} injection | 2^{ème} injection | 3^{ème} injection | |
| Semaine 0 | Semaine 3 | Semaine 6 | |
| MVA gag 10⁷ pfu | PLA/p24 40 µg | | 5 |
| MVA gag 10⁷ pfu | PLA/p24 40 µg | PLA/p24 40 µg | 5 |
| PLA/p24 40 µg | MVA gag 10⁷ pfu | | 5 |
| PLA/p24 40 µg | MVA gag 10⁷ pfu | PLA/p24 40 µg | 5 |
| PLA/p24 40 µg | PLA/p24 40 µg | | 5 |
| PLA/p24 40 µg | PLA/p24 40 µg | PLA/p24 40 µg | 5 |

Les animaux ont été sacrifiés 3 semaines après la dernière injection et le sang et la rate ont été prélevés pour les analyses immunologiques.

### 4. Analyses immunologigues

On a recherché la réponse humorale et la réponse cellulaire comme suit :
- Réponse humorale : on a effectué un prélèvement de sang sur les souris avant leur sacrifice. La présence des anticorps anti-p24 a été déterminée par ELISA. La protéine p24 (produite avec *Escherichia coli* et purifiée par chromatographie d'affinité métal-chélate selon Cheynet V. et al., 1993, Protein Expr Purif, 4(5) : 367-72) a été utilisée en capture et les anticorps spécifiques présents dans le sérum ont été révélés par un anticorps polyclonal anti-souris à titre d'anticorps de détection qui est un anticorps de chèvre anti-IgG de souris AffiniPure conjugué à la peroxydase de raifort (H+L, Jackson Immunoresearch, Cat no 115-035-062). Le titre est l'inverse de la dilution pour laquelle on obtient une absorbance de 0,1 unité DO avec le protocole ELISA utilisé.
- Réponse cellulaire : après sacrifice des souris, les rates ont été prélevées de façon stérile pour préparer une suspension cellulaire. Les analyses suivantes ont été pratiquées sur les suspensions cellulaires obtenues, chaque souris ayant été analysée de manière individuelle ou en pool lorsque la quantité de cellules obtenues n'était pas suffisante pour analyse individuelle.
   *(i) Test CTL :* La suspension cellulaire a été mise en culture en présence d'un peptide de la protéine p24 de 9-mer (AMQMLKETI - SEQ ID N°2) qui correspond à un épitope CTL *H-2K^{d}*-restreint immunodominant, et d'IL-2. Cinq jours plus tard, la population effectrice a été restimulée par des cellules naïves irradiées, chargées avec le peptide. La population cytotoxique effectrice a été récoltée au bout du 7^{ème} jour et l'activité CTL a été mesurée en utilisant comme cibles les cellules P815 (ATCC TIB-64) marquées au ⁵¹Cr.
   *(ii) ELISPOT* : L'ELISPOT permet de déterminer le nombre de cellules sécrétant une cytokine donnée en réponse à un stimulus spécifique. Nous nous sommes intéressés aux cytokines IFN-γ de type Th1 et IL-4 de type Th2. Les suspensions cellulaires obtenues à partir des rates ont été restimulées *in vitro* par le peptide AMQMLKETI pendant 20h pour analyser les réponses de type CDB. Les suspensions cellulaires obtenues à partir des rates ont été restimulées *in vitro* par la protéine p24 (sans endotoxines) pendant 42h pour analyser les réponses de type CD4.
      Les plaques d'ELISPOT 96 puits avec des membranes de PVDF (Multiscreen IP, Millipore) ont été coatées par un anticorps anti-IFN-? ou anti-IL-4. Pendant la restimulation, les suspensions de splénocytes ont été incubées dans ces plaques de manière à capturer les cytokines sécrétées par chaque cellule. Les spots correspondant à chaque cellule sécrétant la cytokine d'intérêt ont été révélés par un anticorps de détection biotinylé spécifique de la cytokine d'intérêt.
   (iii) *Sécrétion des cytokines* : Les splénocytes ont été stimulées pendant 3 jours dans du milieu de culture complet (milieu de culture alpha minimal essential medium (αMEM) contenant 10% sérum de veau foetal, 10 mM HEPES, 5 × 10⁻⁵ M B-mercaptoéthanol, 4 mM L-glutamine, 80 U/ml pénicilline, 80 mg/ml streptomycine, acides amines non-essentiels lx (cat no. 11140-035), sodium pyruvate 1x (cat no. 11360-039) (constituants de Invitrogen, Cergy Pontoise, France)) en présence ou non de l'antigène d'intérêt p24, puis les cytokines sécrétées dans le surnageant de culture ont été dosées par le kit mouse FlowCytomix Th1/Th2 10plex de Bender Medsystems (Cat no. BMS720FF).

### 5. Résultats

### 5.1. La réponse CTL

Le tableau 4 ci-après récapitule les résultats obtenus lors des 3 expériences successives :

**Tableau 4**

| Code | composition vaccinale | Souris analysées en pool (Exp 1, *n*=*5*) | Fréquence des souris avec réponse CTL nb de souris positif/nb de souris analysées en individuel |
|---|---|---|---|
| **A** | MVA gag + MVA gag | Pool négatif | 0/5 |
| **A'** | MVA gag + MVA gag + MVA gag | | 1/5 |
| **B** | MVA gag + PLA/p24 | Pool négatif | 1/10 |
| **B'** | MVA gag + PLA/p24 + PLA/p24 | | 3/10 |
| **C** | PLA/p24 + MVA gag | Pool positif | 2/10 |
| **C'** | PLA/p24 + MVA gag + PLA/p24 | | 1/5 |
| **F** | ADN gag + PLA/p24 | Pool positif | Non effectué |
| **G** | PLA/p24 + ADN gag | Pool positif | Non effectué |
| **D** | PLA/p24 + PLA/p24 | Pool négatif | 0/5 |
| **D'** | PLA/p24 + PLA/p24 + PLA/p24 | | 0/5 |
| **E** | MVA + MVA sauvage | Pool négatif | Non effectué |
| **E'** | MVA + MVA + MVA sauvage | | 0/5 |

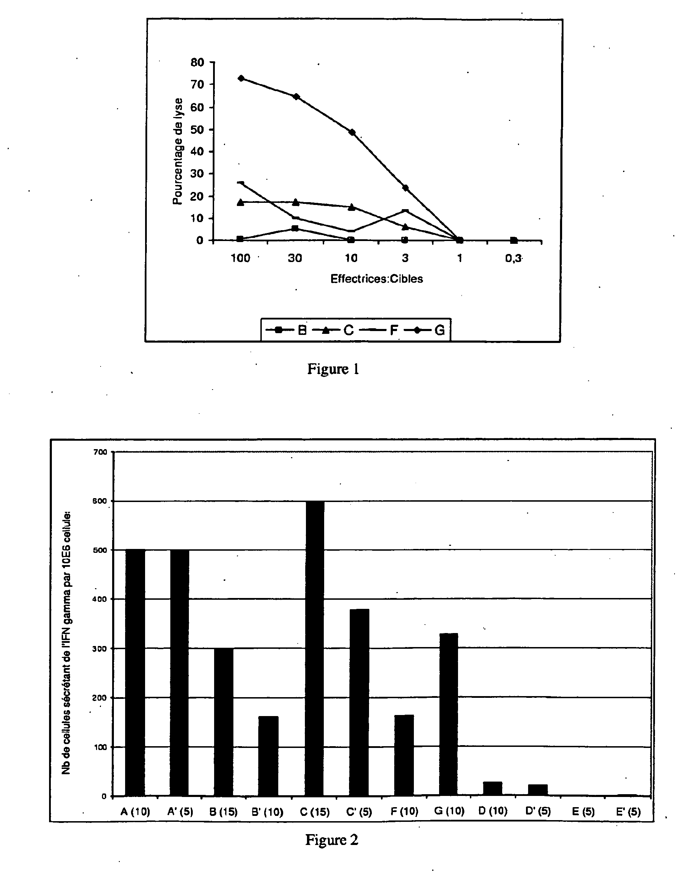

La Figure 1 représente les réponses CTL obtenues lors de l'expérience d'immunisation n°1. Chaque groupe est constitué de 5 souris, les rates ont été poolées avant la réalisation du dosage CTL. La stimulation non spécifique (sans peptide) dans tous les groupes étant de 0, elle n'est pas représentée. De même, les réponses CTL obtenues avec les compositions A, D et E étant de 0, elles ne sont pas représentées. L'abscisse correspond au ratio cellules effectrices (CTL) sur cellules cibles (à lyser). L'axe des ordonnées correspond au pourcentage de lyse moyenne.

Les réponses CTL induites sont de faible intensité avec tous les immunogènes utilisés (MVA, PLA ou combinaisons), à l'exception du groupe G de l'expérience 1 qui a reçu de l'ADN en dernier (Figure 1). L'induction de CTL dans les groupes ADN est normal : l'ADN est optimisé dans ce but, c'est un témoin positif chez la souris mais qui ne marche pas aussi bien chez les primates.

Même si les réponses restent faibles, les groupes contenant à la fois du MVA et du PLA donnent de meilleures réponses CTL que les groupes ayant reçu du MVA seul ou du PLA seul.

Lorsque 2 injections ont été réalisées, dans les groupes ayant reçu 2 fois du MVA ou 2 fois des PLA, il n'y a pas de CTL détecté lors du sacrifice retenu (A et D), alors que dans les groupes ayant reçu à la fois PLA et MVA (B et C) il y a une faible fréquence de CTL. L'ordre d'injection joue aussi un rôle : PLA + MVA (C) donne de meilleurs résultats que MVA + PLA (B).

Lorsque 3 injections ont été réalisées, la fréquence d'induction des réponses reste encore faible. Les réponses CTL ne sont pas améliorées par rapport à deux injections. La stratégie vaccinale utilisant le MVA nécessite 3 injections pour donner les mêmes résultats que les combinaisons de PLA et de MVA.

### 5.2. La réponse ELISPOT IFN-γ contre le peptide Gag AMQ

La figure 2 représente les réponses ELISPOT IFN-γ spécifiques du peptide Gag AMQ. Tous les animaux immunisés lors des 3 expériences successives ont été inclus dans l'analyse. Pour chaque composition vaccinale, le nombre d'animaux ayant reçu cette composition est indiquée entre parenthèses. L'axe des ordonnées représente le nombre moyen de cellules sécrétant de l'IFN-γ de manière spécifique en réponse à une stimulation de 18h par le peptide Gag AMQ sur un million de cellules stimulées. Cette réponse a été déterminée individuellement pour chaque souris. Le nombre présenté sur ce graphique correspond à la moyenne calculée à partir de toutes les souris d'un groupe (n=5, 10 ou 15 selon les groupes).

L'interféron gamma ou de type II est aussi appelé interféron immunitaire car il est sécrété par les lymphocytes T de type CD4+, par les lymphocytes T de type CD8+ et les cellules NK. Il protège les cellules contre les infections virales. Il stimule l'activité phagocytaire des macrophages, leur permettant de tuer les cellules tumorales. Il stimule la maturation des lymphocytes T et B et augmente la production d'anticorps. Il augmente l'expression des molécules HLA de classe I et II par les macrophages. Il s'agit d'une cytokine de type Th1.

Il est possible de classer les compositions vaccinales en plusieurs catégories en fonction des réponses ELISPOT IFN-γ obtenues. Les meilleures réponses sont induites par les compositions PLA + MVA (C) et MVA + MVA (A). Dans ces cas, la troisième injection n'apporte soit aucune amélioration (composition MVA + MVA) (A'), soit peut faire diminuer la réponse observée (composition PLA + MVA) (C'). Les compositions MVA + PLA (B et B') et ADN + PLA (F) permettent de générer des réponses d'intensité intermédiaires, alors que la stratégie utilisant uniquement des PLA (D et D') n'induit que des réponses faibles.

Ainsi, nous avons pu montrer que la composition vaccinale PLA + MVA (C) était au moins aussi bonne, sinon meilleure que la combinaison MVA + MVA (A) pour induire une sécrétion d'IFN-γ spécifique du peptide AMQ.

### 5.3. La réponse ELISPOT IFN-γ contre la protéine p24

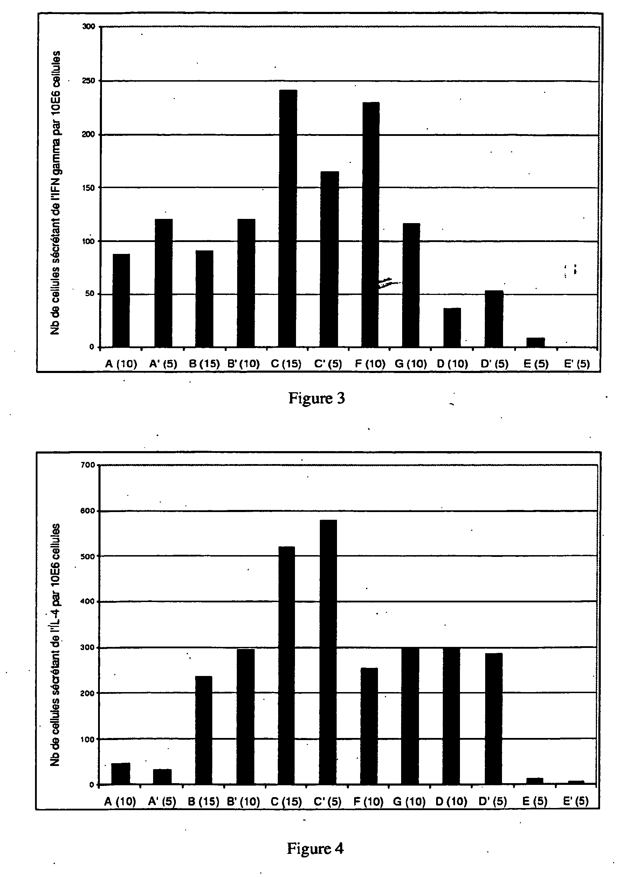

La Figure 3 représente les réponses ELISPOT IFN-γ spécifiques de la protéine p24. Tous les animaux immunisés lors des 3 expériences successives ont été inclus dans l'analyse. Pour chaque composition vaccinale, le nombre d'animaux ayant reçu cette composition est indiquée entre parenthèses. L'axe des ordonnées représente le nombre moyen de cellules sécrétant de l'IFN-γ de manière spécifique en réponse à une stimulation de 42h par la protéine p24 sur un million de cellules stimulées. Cette réponse a été déterminée individuellement pour chaque souris. Le nombre présenté sur ce graphique correspond à la moyenne calculée à partir de toutes les souris d'un groupe (n=5, 10 ou 15 selon les groupes).

Encore une fois, il est possible de classer les compositions vaccinales en plusieurs catégories en fonction des réponses ELISPOT IFN-γ obtenues. Les meilleures réponses sont induites par les compositions PLA + MVA (C et C') et ADN + PLA (F). La troisième injection fait encore diminuer la réponse observée dans le cas de la composition PLA + MVA (C'). Les compositions MVA + PLA (B et B') et MVA+ MVA (A et A') permettent de générer des réponses d'intensité intermédiaires, alors que la stratégie utilisant uniquement des PLA (D et D') n'induit que des réponses faibles.
Ainsi, nous avons pu montrer que la composition vaccinale PLA + MVA était le meilleur choix pour induire une sécrétion d'IFN-γ spécifique de la protéine p24.

### 5.4. La réponse ELISPOT IL-4 contre la protéine p24

La figure 4 représente les réponses ELISPOT IL-4 spécifiques de la protéine p24. Tous les animaux immunisés lors des 3 expériences successives ont été inclus dans l'analyse. Pour chaque composition vaccinale, le nombre d'animaux ayant reçu cette composition est indiquée entre parenthèses. L'axe des ordonnées représente le nombre moyen de cellules sécrétant de l'IL-4 de manière spécifique en réponse à une stimulation de 42h par la protéine p24 sur un million de cellules stimulées. Cette réponse a été déterminée individuellement pour chaque souris. Le nombre présenté sur ce graphique correspond à la moyenne calculée à partir de toutes les souris d'un groupe (n=5, 10 ou 15 selon les groupes).

Encore une fois, il est possible de classer les compositions vaccinales en plusieurs catégories en fonction des réponses ELISPOT IL-4 obtenues. La meilleure réponse est induite par la composition PLA + MVA (C et C'). La troisième injection semble encore une fois non nécessaire car elle n'améliore que de très peu la réponse induite par la composition PLA + MVA (C'). Les compositions MVA + PLA (B et B') et ADN + PLA (F) et PLA + PLA (D et D') permettent de générer des réponses d'intensité intermédiaires, alors que la stratégie utilisant uniquement des MVA n'induit que des réponses faibles (A et A').

Ainsi, nous avons pu montrer que la composition vaccinale PLA + MVA était le meilleur choix pour induire une sécrétion d'IL-4 spécifique de la protéine p24.

### 5.5. Sécrétion des cytokines

L'étude de la sécrétion des cytokines permet de tracer un tableau de l'état d'activation immunitaire de l'animal ayant reçu la composition vaccinale. On peut répartir les cytokines en quatre groupes en fonction de leurs propriétés :
1) Les médiateurs de l'immunité naturelle (IFN-γ, IL-1, IL-6, IL-8 ...)
2) Les activateurs de l'activation, croissance et différenciation lymphocytaire (IL-2, IL-4, IL-5, IL-6, IL-10, TGF-B)
3) Les activateurs non spécifiques de l'inflammation (IFN-γ, IL-5, IL-8)
4) Les activateurs et différenciateurs de leucocytes immatures (IL-3, GM-CSF)
Grâce à l'utilisation d'un kit multiplex, nous avons pu doser la sécrétion de 10 cytokines différentes dans les surnageants de culture des splénocytes obtenues des animaux sacrifiés. Ces cytokines sont : IFN-γ, TNF-α, GM-CSF, IL-4, IL-17, IL-1α, IL-2, IL-5 , IL-6, IL-10.

Aucune des compositions vaccinales testées n'induit une sécrétion significative des cytokines IL-1α et IL-17.

Pour tous les autres cytokines testés nous avons pu mettre en évidence des différences parmi les groupes.

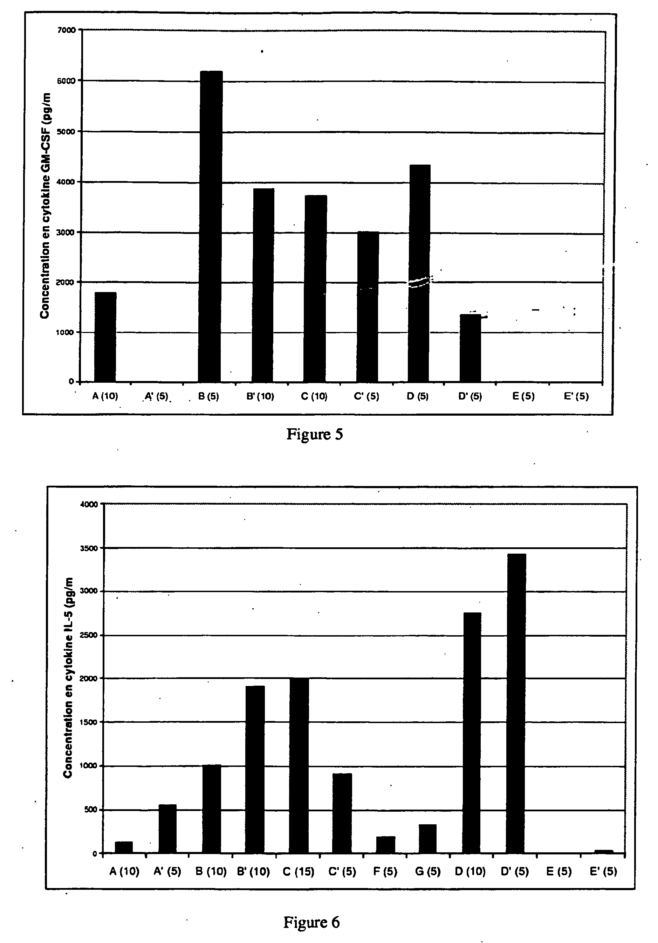

La figure 5 représente la sécrétion de la cytokine GM-CSF spécifique de la protéine p24. Tous les animaux immunisés lors des 3 expériences successives ont été inclus dans l'analyse, lorsque la quantité de cellules disponibles le permettait. Pour chaque composition vaccinale, le nombre d'animaux ayant reçu cette composition est indiquée entre parenthèses. Les cellules obtenues à partir des souris d'un même groupe ont été poolées avant la mise en culture pour le test de sécrétion. L'axe des ordonnées représente la concentration de GM-CSF (en pg/ml) sécrétée dans le milieu de culture en réponse à une stimulation de 3 jours par la protéine p24.

La cytokine GM-CSF (granulocyte-macrohage colony stimulating factor) est un puissant immunostimulateur qui permet d'activer les fonctions immunitaires telles que l'adhésion, la phagocytose, etc. des macrophages, des neutrophiles et des éosinophiles. Comme nous le pouvons voir sur la figure 5, nous avons montré une importante sécrétion de GM-CSF dans les groupes contenant du PLA en association ou non avec le MVA (sauf lorsque le nombre d'injection est augmenté à 3 pour les PLA) alors que les groupes immunisés exclusivement avec du MVA n'en sécrètent pas ou peu.

La figure 6 représente la sécrétion de la cytokine IL-5 spécifique de la protéine p24. Tous les animaux immunisés lors des 3 expériences successives ont été inclus dans l'analyse, lorsque la quantité de cellules disponibles le permettait. Pour chaque composition vaccinale, le nombre d'animaux ayant reçu cette composition est indiquée entre parenthèses. Les cellules obtenues à partir des souris d'un même groupe ont été poolées avant la mise en culture pour le test de sécrétion. L'axe des ordonnées représente la concentration d'IL-5 (en pg/ml) sécrétée dans le milieu de culture en réponse à une stimulation de 3 jours par la protéine p24.

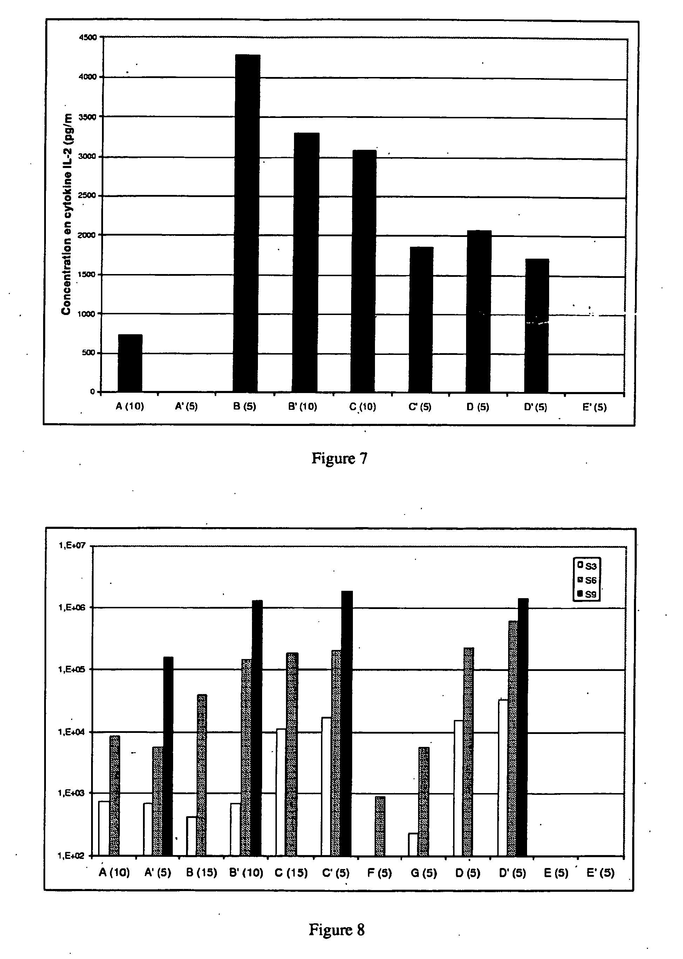

Pour tous les cytokines de type Th2 testées comme IL-4, IL-5, IL-6 et IL-10, les profils de sécrétion sont très similaires à celui présenté en figure 6 qui correspond à la sécrétion de l'IL-5. Aucune sécrétion n'est observée dans les groupes ayant reçu du MVA sauvage (c'est normal, c'est un témoin négatif). De faibles concentrations sont obtenues pour les groupes MVA gag, 2 ou 3 injections, et les groupes associant l'ADN et le PLA. Des concentrations moyennes sont obtenues pour les groupes de souris immunisées par les compositions PLA et MVA. Les concentrations les plus fortes sont obtenues dans les groupes ayant reçu uniquement du PLA. La sécrétion de cytokines est donc orientée plus ou moins vers les cytokines de type Th2 en fonction de la composition vaccinale :
- on observe une réponse majoritairement de type Th2 pour les groupes immunisés uniquement avec les PLA (la sécrétion d'IFN-gamma de d'IL-2, cytokines de type Th1 est faible (figure 7),
- on observe une réponse non polarisée, à la fois Th1 et Th2 pour les groupes immunisés avec les combinaisons PLA et MVA.
- pour les groupes MVA, la sécrétion des cytokines de type Th2 est faible.

Notons aussi que les 4 cytokines Th2 dosées ne sont pas sécrétées à la même concentration. C'est l'IL-4 et l'IL-10 qui sont sécrétées à des taux élevés. Le tableau suivant récapitule les concentrations moyennes des différentes cytokines observées dans les groupes ayant reçu les compositions PLA et MVA.

**Tableau 5**

| Cytokine | IL-4 | IL-5 | IL-6 | IL-10 |
|---|---|---|---|---|
| Conc moyenne (pg/ml) | 2600 | 1460 | 260 | 2660 |

L'interleukine 4, IL-4, est sécrétée par les lymphocytes T de type CD4+. Elle augmente la croissance et la différenciation des lymphocytes B préalablement activés, favorise la production d'IgE qui joue un rôle important dans les réactions d'hypersensibilité immédiate. Elle active aussi les macrophages.

L'interleukine 5, IL-5, stimule la croissance, la différenciation et l'activité des éosinophiles qui jouent un rôle important dans la lutte contre les infections parasitaires. L'augmentation du nombre des éosinophiles n'est pas seulement un signe d'atteinte parasitaire mais aussi un moyen de défense. L'IL-5 induit la prolifération des lymphocytes B et leur sécrétion d'immunoglobulines. Elle active les lymphocytes T cytotoxiques. Son utilisation dans le traitement de certaines maladies parasitaires peut être envisagée.

L'interleukine 6, IL-6, stimule la croissance et la différenciation des lymphocytes B et augmente la génération des plaquettes. Elle provoque, par activation des hépatocytes, la sécrétion des protéines de l'inflammation comme le fibrinogène et la protéine C réactive. Elle a un rôle pro-inflammatoire. Elle a un effet cytotoxique vis-à-vis de certaines tumeurs.

La figure 7 représente la sécrétion de la cytokine IL-2 spécifique de la protéine p24. Tous les animaux immunisés lors des 3 expériences successives ont été inclus dans l'analyse, lorsque la quantité de cellules disponibles le permettait. Pour chaque composition vaccinale, le nombre d'animaux ayant reçu cette composition est indiquée entre parenthèses. Les cellules obtenues à partir des souris d'un même groupe ont été poolées avant la mise en culture pour le test de sécrétion. L'axe des ordonnées représente la concentration d'IL-2 (en pg/ml) sécrétée dans le milieu de culture en réponse à une stimulation de 3 jours par la protéine p24.

L'IL-2 est un facteur de croissance pour les lymphocytes T. Elle active leur transformation en lymphocytes T cytotoxiques de type CD8+ qui sécrètent l'interféron α, lequel stimule les macrophages à libérer le TNFα et le TGFα (transforming growth factor α). L'IL-2 stimule la croissance et l'activité cytolytique des cellules NK (natural killer). Elle stimule la maturation des lymphocytes B et la synthèse d'anticorps. C'est une cytokine de type Th1.

Il est possible de classer les compositions vaccinales en plusieurs catégories en fonction de la sécrétion d'IL-2, spécifique de la protéine p24. La meilleure réponse est induite par la combinaison MVA + PLA (B), puis PLA + MVA (C), PLA seul (D) et MVA seul (A).

### 5.6. La réponse humorale spécifique de la protéine p24

La figure 8 représente une cinétique d'apparition des réponses humorales spécifiques de la protéine p24. Tous les animaux immunisés lors des 3 expériences successives ont été inclus dans l'analyse. Pour chaque composition vaccinale, le nombre d'animaux ayant reçu cette composition est indiquée entre parenthèses. L'axe des ordonnées représente le titre en IgG anti-p24 mesuré par ELISA de capture p24 ; l'échelle de cet axe est logarithmique. Le titre en anticorps a été déterminé individuellement pour chaque souris. Le nombre présenté sur ce graphique correspond à la moyenne géométrique calculée à partir de toutes les souris d'un groupe (n=5, 10 ou 15 selon les groupes). S3 = semaine 3, S6 = semaine 6, S9 = semaine 9.

En ce qui concerne les réponses humorales anti-p24, pour la première fois dans cette série d'expérience, augmenter le nombre d'injections permet d'améliorer de façon significative la réponse immune mesurée. En effet, quel que soit le groupe étudié, passer de 2 à 3 injections d'immunogène augmente environ d'un log la réponse anti-p24 (sauf pour le groupe PLA seul où l'augmentation est moindre mais tout de même significative).

Pour les groupes ayant reçu 3 injections, toutes les combinaisons contenant du MVA et du PLA ou le PLA seul induisent un titre élevé (10^{E}6), supérieur d'un log au titre induit par du MVA seul.

De façon similaire, pour les groupes ayant reçu 2 injections, toutes les compositions contenant du MVA et du PLA ou le PLA seul induisent un titre élevé, supérieur d'un log au titre induit par du MVA seul.

Changer une injection de PLA en MVA dans un schéma de vaccination ne nuit pas aux titres en anticorps obtenus mais permet d'élargir les réponses cellulaires par rapport à celles qui sont générées uniquement par un vecteur PLA.

### Conclusion :

En fonction du type de réponse analysée, la meilleure composition n'est pas toujours la même, certaines compositions obtenant spécifiquement de très bonnes réponses.

Cependant, pour obtenir une réponse immune large et intense, la meilleure composition est la composition PLA + MVA.

### Exemple 2: Immunisation de souris avec la composition vaccinale microparticules PLA/p24 et l'Adénovirus Gag

### 1. Modèle animal

Les expériences d'immunisation ont été réalisées chez des souris BALB/c, (H-2K^{d}) femelles âgées de 6 à 8 semaines au moment de la première immunisation.

### 2. Immunogènes administrés

Dans cette série d'expériences ont été utilisés seuls ou en combinaison les immunogènes suivants :
- les microparticules PLA/p24 préparées selon le protocole déplacement de solvant de la demande de brevet WO2005/02787.
- L'Adénovirus type 5 Gag qui est recombinant pour le gène gag du virus VIH-1, codant pour la protéine p24. (Généthon)
- L'Adénovirus type 5 eGFP qui est recombinant pour le gène codant pour la enhanced Green Fluorescent Protein (Généthon). Il s'agit d'un témoin négatif qui sert à mesurer si le vecteur viral induit une réponse immune non spécifique lors des immunisations.
- l'ADN gag. Le gène gag (SEQ ID N°1) a été cloné dans le plasmide d'expression eucaryote phCMV (plasmide non commercial). Les préparations d'ADN sans endotoxines et en grande quantité ont été réalisées avec le kit de Gigaprep endotoxine-free de Macherey-Nagel. L'ADN sert de contrôle positif, car il est connu que chez la souris l'administration par voie intramusculaire d'ADN nu permet d'induire une bonne réponse cytotoxique (CTL).

### 3. Immunisations

30 souris ont reçu 2 doses des immunogènes décrits dans le point 2 ci-dessus à 0 et 3 semaines. Les compositions vaccinales reçues par chaque groupe de souris sont indiquées dans le tableau 6 ci-après. Toutes les injections ont été réalisées par voie intramusculaire pour l'adénovirus et l'ADN et par voie sous-cutanée pour les PLA.

**Tableau 6**

| Dose | Dose | Nombre de souris | Composition |
|---|---|---|---|
| 1^{ère} injection | 2^{ème} injection | | |
| semaine 0 | semaine 3 | | |
| Ad5 gag 10⁸ ip | Ad5 gag 10⁸ ip | 5 | H |
| Ad5 gag 10⁸ ip | PLA/p24 40 µg | 5 | I |
| PLA/p24 40 µg | Ad5 gag 10⁸ ip | 5 | J |
| Ad5 gag 10⁷ ip | Ad5 gag 10⁷ ip | 5 | H' |
| Ad5 gag 10⁷ ip | PLA/p24 40 µg | 5 | I' |
| PLA/p24 40 µg | Ad5 gag 10⁷ ip | 5 | J' |

Les animaux ont été sacrifiés 3 semaines après la dernière injection et le sang et la rate ont été prélevés pour les analyses immunologiques.

### 4. Analyses immunologiques

Réalisées suivant les mêmes protocoles que dans l'exemple 1. De plus, nous avons mesuré les réponses humorales dirigées contre l'adénovirus5 en utilisant une technique ELISA. Le protocole est identique à celui utilisé pour l'ELISA anti-p24, sauf qu'un lysat d'adénovirus est utilisé en capture.

### 5. Résultats

### 5.2. Réponses CTL

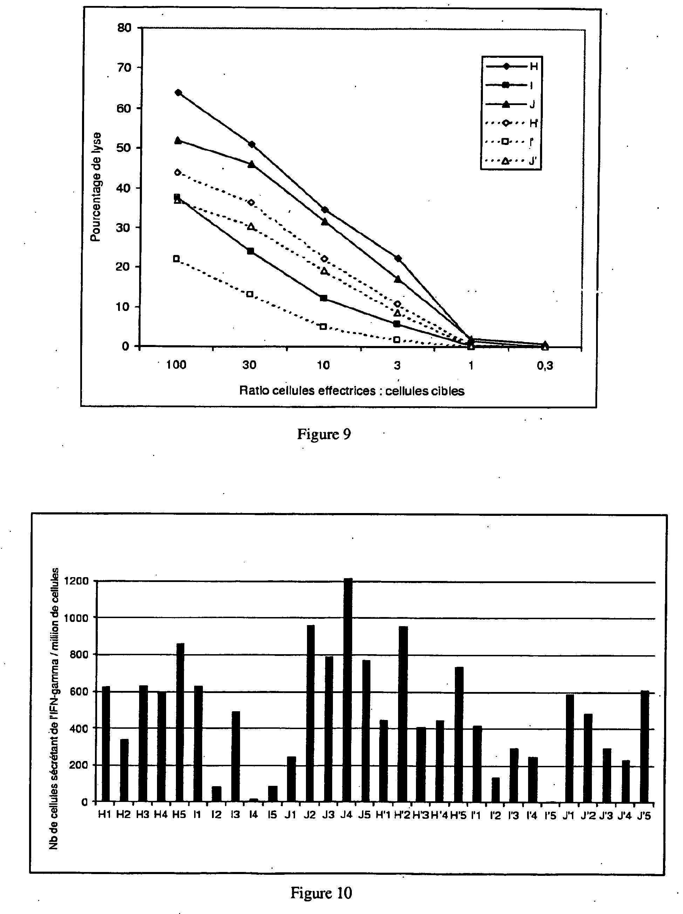

La figure 9 représente les réponses CTL obtenues lors de l'expérience d'immunisation n°2. Chaque groupe est constitué de 5 souris, analysée individuellement. La stimulation non spécifique (sans peptide) dans tous les groupes étant de 0, elle n'est pas représentée. L'abscisse correspond au ratio cellules effectrices (CTL) sur cellules cibles (à lyser). L'axe des ordonnées correspond au pourcentage de lyse moyenne ; il s'agit de la moyenne arithmétique calculée à partir de toutes les souris d'un groupe (n=5).

Dans tous les groupes 5 souris sur 5 ont développé une réponse CTL, sauf une souris du groupe Ad5gag 10⁷ + PLA.

On observe la même chose pour les 2 doses de virus testées : les combinaisons Ad + Ad (H et H') et PLA + Ad (J et J') induisent de meilleures réponses CTL que la combinaison Ad + PLA (I et I').

### 5.3. La réponse ELISPOT IFN-γ contre le peptide Gag AMQ

La Figure 10 représente les réponses ELISPOT IFN-γ spécifiques du peptide Gag AMQ. L'axe des ordonnées représente le nombre moyen de cellules sécrétant de l'IFN-γ de manière spécifique en réponse à une stimulation de 18h par le peptide Gag AMQ sur un million de cellules stimulées. Cette réponse a été déterminée individuellement pour chaque souris (numérotée de 1 à 5 pour chaque souris, ainsi H1 correspond à la souris 1 ayant reçu la composition H Ad5 gag 10⁸ ip et Ad5 gag 10⁸ ip, etc. Le nombre présenté-sur ce graphique correspond à une moyenne de triplicats.

On peut noter d'après la figure 10 que :
- à 10⁸ : la réponse PLA + Ad > Ad + Ad > Ad + PLA
- à 10⁷ : la réponse Ad + Ad > ou = PLA + Ad > Ad + PLA. La différence entre les compositions Ad + Ad et PLA + Ad est peu flagrante. Globalement la réponse ELISPOT IFN-gamma est en concordance avec le test CTL : Les compositions PLA + Ad et Ad + Ad sont plus efficaces que Ad + PLA.

### 5.4. Sécrétion de cytokines

Comme dans l'exemple 1, nous avons testé la sécrétion de 10 cytokines dans le surnageant. Parmi celles-ci, l'IL-5, l'IL-6, l'IL-17 et l'IFN-γ n'étaient pas sécrétées ou sécrétées à des concentrations infinitésimales. Quant au GM-CSF, TNF-α et IL-1α, leur sécrétion n'était pas spécifique de la protéine p24 par la technique de dosage utilisée. Il est très probable que c'est l'immunisation par l'adénovirus qui provoque la sécrétion de ces cytokines. Si une sécrétion spécifique de la protéine p24 existe, elle est noyée dans celle provoquée par l'adénovirus.

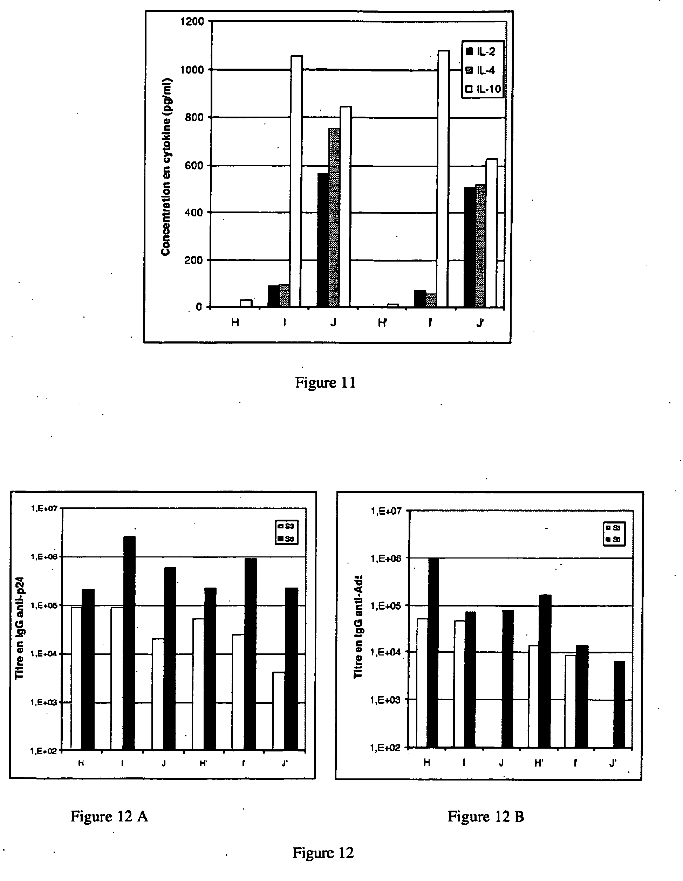

Pour 3 autres cytokines, à savoir IL-2 (Th1), ainsi que l'IL-4 et l'IL-10 (Th2), nous avons pu mesurer la sécrétion spécifique de la protéine p24. Les résultats sont présentés dans la figure 11.

La figure 11 représente la sécrétion des cytokines IL-2, IL-4 et IL-10 spécifique de la protéine p24. Chaque composition vaccinale a été administrée à 5 souris. Les cellules obtenues à partir des souris d'un même groupe ont été poolées avant la mise en culture pour le test de sécrétion. L'axe des ordonnées représente la concentration de chaque cytokine (en pg/ml) sécrétée dans le milieu de culture en réponse à une stimulation de 3 jours par la protéine p24.

Les résultats sur la figure 11 montrent que :
- la composition Ad5 gag + Ad5 gag (H et H')ne donne aucune sécrétion d'IL-2, IL-4 ou IL-10.
- seule la composition PLA + Ad5 gag (J et J') donne une sécrétion de IL-2 et IL-4.
- les compositions PLA + Ad5 gag (J et J') et Ad5 gag + PLA (I et I') permettent une sécrétion de IL-10.

La composition Ad + Ad (H et H') induit une réponse cytokine plutôt polarisée Th1 (mesurée par ELISPOT IFN-gamma)

La composition PLA + Ad 5 (J et J') induit une réponse eytokine non polarisée, puisque à la fois des cytokines Th1 comme IFN-gamma (par ELISPOT) et l'IL-2, et des cytokines Th2 comme l'IL-4 et L'IL-10 sont détectées.

### 5.5. Réponses humorales

La figure 12 représente la cinétique d'apparition des réponses humorales. La figure 12(A) représente la réponse anticorps dirigée contre la protéine p24 et la figure 12(B) représente la réponse anticorps dirigée contre l'adénovirus. Chaque composition vaccinale a été administrée à 5 souris. L'axe des ordonnées représente le titre en IgG mesuré par ELISA ; l'échelle de cet axe est logarithmique. Le titre en anticorps a été déterminé individuellement pour chaque souris. Le nombre présenté sur ce graphique correspond à la moyenne géométrique calculée à partir de toutes les souris d'un groupe (n=5). S3 = semaine 3, S6 = semaine 6.

La réponse anti-adénovirus (ou réponse anti-vecteur) est d'environ 1 log de moins dans les groupes ayant reçu les compositions Ad+PLA (I et I') et PLA+Ad (J et J') que pour Ad+Ad (H et H').

Les compositions selon l'invention permettent donc de diminuer l'immunité anti-adénovirus.

### Exemple 3: Comparaison des compositions vaccinales PLA/p24 + MVAgag et Alum/p24 + MVAgag chez la souris

### 1. Modèle animal

Les expériences d'immunisation ont été réalisées chez des souris BALB/c (H-2^{d}) femelles âgées de 6 à 8 semaines au moment de la première immunisation.

### 2. Immunogènes administrés

Dans cette série d'expériences ont été utilisés seuls ou en combinaison les immunogènes suivants :
- les microparticules PLA/p24 préparées selon le protocole déplacement de solvant et d'adsorption de la demande de brevet WO 2005/02787.
- alum/p24 : l'alum (Imject Alum, Pierce, No de catalogue 77161) est mélangé volume pour volume (1:1) avec la protéine p24 diluée dans du PBS. On administre une dose de 40 µg de p24 par injection et par animal.
- le virus MVA gag (Modified Vaccinia Virus Ankara, virus de la vaccine atténué, souche Ankara) qui est recombinant pour le gène gag du virus VIH-1, codant pour la protéine p24. Le virus recombinant a été construit à Transgene, comme indiqué dans l'exemple 1.
- l'ADN gag. Le gène gag de séquence SEQ ID N°1 a été cloné dans le plasmide d'expression eucaryote phCMV. Les préparations d'ADN sans endotoxines et en grande quantité ont été réalisées avec le kit de Gigaprep endotoxine-free de Macherey-Nagel. L'ADN sert de contrôle positif, car il est connu que chez la souris l'administration par voie intramusculaire d'ADN nu permet d'induire une bonne réponse cytotoxique (CTL).

### 3. Immunisations

30 souris ont reçu 2 doses des immunogènes décrits dans le point 2 ci-dessus à 0 et 3 semaines. Les compositions vaccinales reçues par chaque groupe de souris sont indiquées dans le tableau 7 ci-après. Toutes les injections ont été réalisées par voie sous-cutanée sauf pour l'ADN qui a été administré par voie intramusculaire.

**Tableau 7**

| Dose | Dose | Nombre de souris | Composition |
|---|---|---|---|
| 1^{ère} injection | 2^{ème} injection | | |
| semaine 0 | semaine 3 | | |
| MVA gag 10⁷ pfu | PLA/p24 40 µg | 5 | B |
| PLA/p24 40 µg | MVA gag 10⁷ pfu | 5 | C |
| MVA gag 10⁷ pfu | Alum/p24 40 µg | 5 | K |
| Alum/p24 40 µg | MVA gag 10⁷ pfu | 5 | L |
| PLA/p24 40 µg | ADN gag 5 µg (IM) | 5 | G |
| Alum/p24 40 µg | ADN gag 5 µg (IM) | 5 | M |

Pfu = plaque forming units, c'est l'unité de mesure de la dose de MVA utilisée. Le titre viral est exprimé en pfu selon la technique de dosage du stock viral utilisée.

Les animaux ont été sacrifiés 6 semaines après la première injection et le sang et la rate ont été prélevés pour les analyses immunologiques.

### 4. Analyses immunologiques

Réalisées suivant les mêmes protocoles que dans l'exemple 1. De plus, nous avons mesuré les réponses humorales dirigées contre le MVA en utilisant une technique ELISA. Le protocole est identique à celui utilisé pour l'ELISA anti-p24, sauf qu'un lysat de MVA est utilisé en capture.

### 5. Résultats

### 5.1. La réponse ELISPOT IFN-γ contre le peptide Gag AMQ

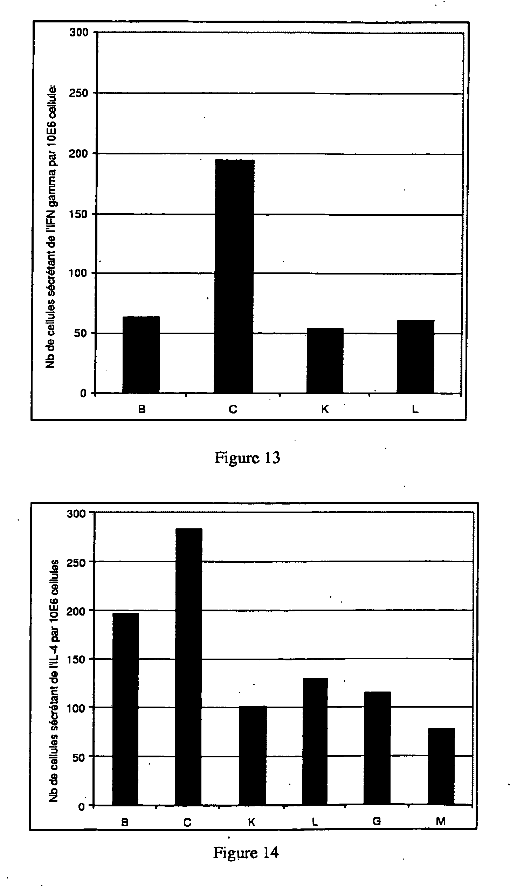

La figure 13 représentent les réponses ELISPOT IFN-γ spécifiques du peptide Gag AMQ. L'axe des ordonnées représente le nombre moyen de cellules sécrétant de l'IFN-γ de manière spécifique en réponse à une stimulation de 18h par le peptide Gag AMQ sur un million de cellules stimulées. Cette réponse a été déterminée individuellement pour chaque souris. Le nombre présenté sur ce graphique correspond à la moyenne calculée à partir de toutes les souris d'un groupe (n=5).

Les réponses ELISPOT IFN-γ obtenues lors de cette expérience sont plus faibles que d'habitude. Cette baisse est observée à la fois pour les groupes testés et les groupes contrôles, et est liée au déroulement de l'expérience. De ce fait, comme nous cherchons à comparer les différentes combinaisons entre elle, cette expérience reste interprétable et montre clairement que la composition PLA/p24 + MVA gag (C) induit une meilleure sécrétion d'IFN-γ mesurée par ELISPOT que les autres groupes testées, à savoir MVA gag + PLA/p24 (B), MVA gag + Alum/p24 (K), Alum/p24 + MVA gag (L).

### 5.2. La réponse ELISPOT IL-4 contre la protéine p24

La figure 14 représente les réponses ELISPOT IL-4 spécifiques de la protéine p24. L'axe des ordonnées représente le nombre moyen de cellules sécrétant de l'IFN-γ de manière spécifique en réponse à une stimulation de 42h par la protéine p24 sur un million de cellules stimulées. Cette réponse a été déterminée individuellement pour chaque souris. Le nombre présenté sur ce graphique correspond à la moyenne calculée à partir de toutes les souris d'un groupe (n=5).

Pour l'induction d'une réponse ELISPOT de type IL-4, le meilleur immunogène est constitué par l'association MVAgag et PLA/p24 (B et C). Quelque soit l'ordre d'administration de cette composition, les réponses IL-4 obtenues sont supérieures à celles induites par les compositions MVA gag + Alum/p24 (quel que soit l'ordre) (K et L), PLA/p24 + ADNgag (G) et Alum/p24 + ADNgag (M). Si l'on s'intéresse maintenant à l'ordre d'administration des produits, c'est toujours la composition PLA/p24 + MVAgag (C) qui donne de meilleurs résultats que la combinaison inverse, MVAgag + PLA/p24 (B).

### 5.3. La réponse humorale spécifique de la protéine p24

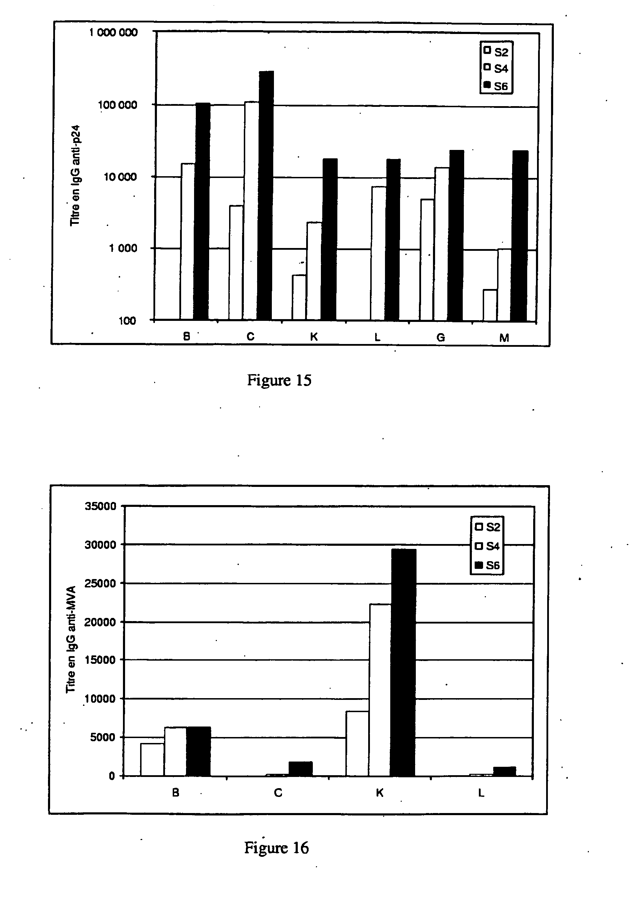

La figure 15 représente la cinétique d'apparition des réponses humorales spécifiques de la protéine p24. L'axe des ordonnées représente le titre en IgG anti-p24 mesuré par ELISA de capture p24 ; l'échelle de cet axe est logarithmique. Le titre en anticorps a été déterminé individuellement pour chaque souris. Le nombre présenté sur ce graphique correspond à la moyenne géométrique calculée à partir de toutes les souris d'un groupe (n=5). S2 = semaine 2, S4 = semaine 4, S6 = semaine 6.

Pour l'induction dune réponse anticorps spécifique de la protéine p24, le meilleur immunogène est constitué encore une fois par l'association MVAgag et PLA/p24 (B et C). Quelque soit l'ordre d'administration de cette composition, les titres d'IgG anti-P4 obtenus sont de 0,6 à 1,2 log plus élevées à semaine 6 que celles induites par les combinaisons MVA gag + Alum/p24 (quel que soit l'ordre) (K et L), PLA/p24 + ADNgag (G) et Alum/p24 + ADNgag (M). Si l'on s'intéresse maintenant à l'ordre d'administration des produits, c'est toujours la combinaison PLA/p24 + MVAgag (C) qui donne de meilleurs résultats que la combinaison inverse, MVAgag + PLA/p24 (B), mais seulement de 0,4 log.

### 5.4. La réponse humorale spécifique du virus MVA

La figure 16 représente la cinétique d'apparition des réponses humorales spécifiques du virus MVA. L'axe des ordonnées représente le titre en IgG anti-MVA mesuré par ELISA de capture MVA. Le titre en anticorps a été déterminé individuellement pour chaque souris. Le nombre présenté sur ce graphique correspond à la moyenne géométrique calculée à partir de toutes les souris d'un groupe (n=5). S4 = semaine 4, S6 = semaine 6.

Dans les groupes PLA/p24 + MVAgag (C) et Alum/p24 + MVAgag (L), le MVA a été administré à semaine 3. Comme nous pouvons le voir sur la figure 16, la réponse anti-MVA n'a pas eu le temps de se mettre en place au moment de l'analyse, à semaine 6. Par contre dans les groupes ayant reçu l'injection de MVA en premier, il est possible de mesurer la réponse anti-vecteur. De façon étonnante, dès 4 semaines, la réponse anti-MVA est bien plus faible dans le groupe MVAgag + PLA/p24 (B) que dans le groupe MVAgag + Alum/p24 (K). De plus avec la combinaison MVAgag + PLA/p24, le titre anti-MVA n'augmente pas dans le temps, ce qui n'est pas le cas avec la combinaison MVAgag + Alum/p24.

En association avec le MVA, les PLA permettent de limiter la réponse anticorps anti-MVA alors que ce n'est pas le cas de l'association MVA + Alum. Il s'agit d'une propriété très intéressante et très utile pour des applications d'immunisation et de vaccination.

### Conclusion :

Les compositions selon l'invention PLA/p24 et MVAgag permettent d'induire de meilleures réponses cellulaires et humorales que les compositions Alum/p24 et MVAgag. De plus, elles permettent de limiter la réponse anti-vecteur.

### Exemple 4: Administration des compositions vaccinales PLA/p24 et MVAgag par voie intranasale

### 1. Modèle animal

Les expériences d'immunisation ont été réalisées chez des souris BALB/c (H-2^{d}) femelles âgées de 6 à 8 semaines au moment de la première immunisation.

### 2. Immunogènes administrés

Dans cette série d'expériences ont été utilisés seuls ou en combinaison les immunogènes suivants :
- les microparticules PLA/p24 préparées selon le protocole déplacement de solvant et d'adsorption de la demande de brevet W02005/027871.
- le virus MVA gag (Modified Vaccinia Virus Ankara, virus de la vaccine atténué, souche Ankara) qui est recombinant pour le gène gag du virus VIH-1, codant pour la protéine p24. Le virus recombinant a été construit à Transgene, comme indiqué precédemment.

### 3. Immunisations

10 souris ont reçu 3 doses des immunogènes décrits dans le point 2 ci-dessus à 0, 10 et 20 jours. Les compositions vaccinales reçues par chaque groupe de souris sont indiquées dans le tableau 8 ci-après. Toutes les injections ont été réalisées par voie intranasale.

**Tableau 8**

| Dose | Dose | Dose | Nombre de souris |
|---|---|---|---|
| 1^{ère} injection | 2^{ème} injection | 3^{ème} injection | |
| jour 0 | jour 10 | jour 20 | |
| PLA/p24 20 µg | PLA/p24 20 µg | MVA gag 10⁷ pfu | 5 |
| MVA gag 10⁷ pfu | MVA gag 10⁷ pfu | PLA/p24 20 µg | 5 |

Pfu = plaque forming units, c'est l'unité de mesure de la dose de MVA utilisée. Le titre viral est exprimé en pfu selon la technique de dosage du stock viral utilisée.

Les animaux ont été sacrifiés 32 jours après la première injection. Le sang, les sécrétions vaginales, les fèces, la rate et l'appareil génital (vagin) ont été prélevés pour les analyses immunologiques.

### 4. Analyses immunologiques

Réalisées suivant les mêmes protocoles que dans l'exemple 1.

### 5. Résultats

### Récapitulation des données expérimentales

**Tableau 9**

| Combinaisons vaccinales | PLA/p24, PLA/p24, MVAgag | | MVAgag, MVAgag, PLA/p24 | |
|---|---|---|---|---|
| Nombre de souris ayant développé une réponse CIL | 2/5 | | 2/5 | |
| % moyen de lyse des souris CTL+ | 50% | | 45% | |
| | | | | |
| L'ELISPOT mesure le nombre de cellules sécrétant la cytokine par 10^{E}6 cellules analysées | | | | |
| ELISPOT IFN-γ, AMQ, sang | 155 | | 79 | |
| ELISPOT IFN-γ, p24, sang | 146 | | 94 | |
| ELISPOT IL-4, AMQ, sang | 50 | | 10 | |
| ELISPOT IL-4, p24, sang | 126 | | 32 | |
| ELISPOT IFN-γ, AMQ, vaginal | 1550 | | 754 | |
| ELISPOT IL-4, AMQ, vaginal | 0 | | 0 | |
| ELISPOT IL-4, p24, vaginal | 0 | | 0 | |
| | | | | |
| Titre en IgG anti-p24 du sérum (déterminé par ELISA) | 68000 | | 200 000 | |
| Titre en IgA anti-p24 des sécrétions vaginales (déterminé par ELISA) | 18 | | 8 | |
| Titre en IgG anti-p24 des sécrétions vaginales (déterminé par ELISA) | 124 | | 616 | |
| IgA anti-p24 dans les fèces (Absorbance à 450 | J20 | 0,20 | J20 | 0,18 |
| nm, déterminée par ELISA) | J32 | 0,02 | J32 | 0,03 |
| IgG anti-p24 dans les fèces (Absorbance à 450 | J20 | 0,17 | J20 | 0,17 |
| nm, déterminée par ELISA) | J32 | 0,69 | J32 | 0,60 |

L'administration des combinaisons PLA/p24 et MVAgag par voie intranasale permet d'induire à la fois une réponse immune systémique mesurée dans les prélèvements de sang et de rate, et une réponse immune périphérique au niveau des muqueuses, en particulier au niveau du tractus uro-génital. A chaque fois, les deux bras du système immunitaire sont activés : on a pu mettre en évidence au niveau de ces deux sites des réponses immunes cellulaires et humorales dirigées contre la protéine p24. La capacité à induire une large réponse périphérique au niveau du tractus uro-génital constitue un atout considérable pour les combinaisons PLA/p24 et MVAgag dans les applications vaccinales à but prophylactique ou thérapeutique.

### Exemple 5: Immunisation de souris avec la combinaison vaccinale microparticules PLA/beta-gal et virus MVA beta-gal

### 1. Modèle animal

Les expériences d'immunisation ont été réalisées chez des souris BALB/c (H-2^{d}) femelles âgées de 6 à 8 semaines au moment de la première immunisation.

### 2. Immunogènes administrés

Dans cette série d'expériences ont été utilisés seuls ou en combinaison les immunogènes suivants :
- les microparticules PLA/beta-gal préparées selon le protocole déplacement de solvant décrit dans la demande de brevet WO2005/027871.
- le virus MVA beta-gal (Modified Vaccinia Virus Ankara, virus de la vaccine atténué, souche Ankara) qui est recombinant pour le gène lacZ de *E. coli,* codant pour l'enzyme beta-galactosidase (beta-gal). Le virus recombinant a été construit à Transgene, et produit et purifié comme décrit précédemment.

### 3. Immunisations

35 souris ont reçu 2 doses des immunogènes décrits dans le point 2 ci-dessus à 0 et 3 semaines. Les combinaisons vaccinales reçues par chaque groupe de souris sont indiquées dans le tableau 10 suivant. Toutes les injections ont été réalisées par voie sous-cutanée.

**Tableau 10**

| Code | Dose | Dose | Nombre de souris |
|---|---|---|---|
| | 1^{ère} injection | 2^{ème} injection | |
| | semaine 0 | semaine 3 | |
| M | PLA/beta-gal 40 µg | PLA/beta-gal 40 µg | 5 |
| N | MVA beta-gal 10⁷ pfu | MVA beta-gal 10⁷ pfu | 5 |
| O | PLA/beta-gal 40 µg | MVA beta-gal 10⁷ pfu | 5 |
| P | MVA beta-gal 10⁷ pfu | PLA/beta-gal 40 µg | 5 |
| Q | PLA/beta-gal 40 µg + MVA beta-gal 10⁷ pfu | | 5 |
| R | | PLA/beta-gal 40 µg + MVA beta-gal 10⁷ pfu | 5 |
| S | PLA/beta-gal 40 µg + MVA beta-gal 10⁷ pfu | PLA/beta-gal 40 µg + MVA beta-gal 10⁷ pfu | 5 |

Pfu = plaque forming units, c'est l'unité de mesure de la dose de MVA utilisée. Le titre viral est exprimé en pfu selon la technique de dosage du stock viral utilisée.

Les animaux ont été sacrifiés 6 semaines après la première injection et le sang et la rate ont été prélevés pour les analyses immunologiques.

### 4. Analyses immunologiques

On a recherché la réponse humorale et la réponse cellulaire comme suit :
- Réponse cellulaire : après sacrifice des souris, les rates ont été prélevées de façon stérile pour préparer une suspension cellulaire. L'ELISPOT permet de déterminer le nombre de cellules sécrétant une cytokine donnée en réponse à un stimulus spécifique. Nous nous sommes intéressés à la cytokine IFN-γ de type Th1. Les suspensions cellulaires obtenues à partir des rates ont été restimulées *in vitro* par le peptide TPH (TPHPARIGL- SEQ ID N°3) pendant 20h pour analyser les réponses de type CD8. Les plaques d'ELISPOT 96 puits avec des membranes de PVDF (Multiscreen IP, Millipore) ont été coatées par un anticorps anti-IFN-γ. Pendant la restimulation, les suspensions de splénocytes ont été incubées dans ces plaques de manière à capturer les cytokines sécrétées par chaque cellule. Les spots correspondant à chaque cellule sécrétant la cytokine d'intérêt ont été révélés par un anticorps de détection biotinylé spécifique de la cytokine d'intérêt.
- Réponse humorale : on a effectué un prélèvement de sang sur les souris avant leur sacrifice. La présence des anticorps anti-beta-gal a été déterminée par ELISA. La protéine beta-gal a été utilisée en capture et les anticorps spécifiques présents dans le sérum ont été révélés par un anticorps polyclonal anti-souris à titre d'anticorps de détection qui est un anticorps de chèvre anti-IgG de souris AffiniPure conjugué à la peroxydase de raifort (H+L, Jackson Immunoresearch, Cat no 115-035-062). Le titre est l'inverse de la dilution pour laquelle on obtient une absorbance de 0,1 unité DO avec le protocole ELISA utilisé.

### 5. Résultats

### 5.1. La réponse ELISPOT IFN-γ contre le peptide beta-gal TPH

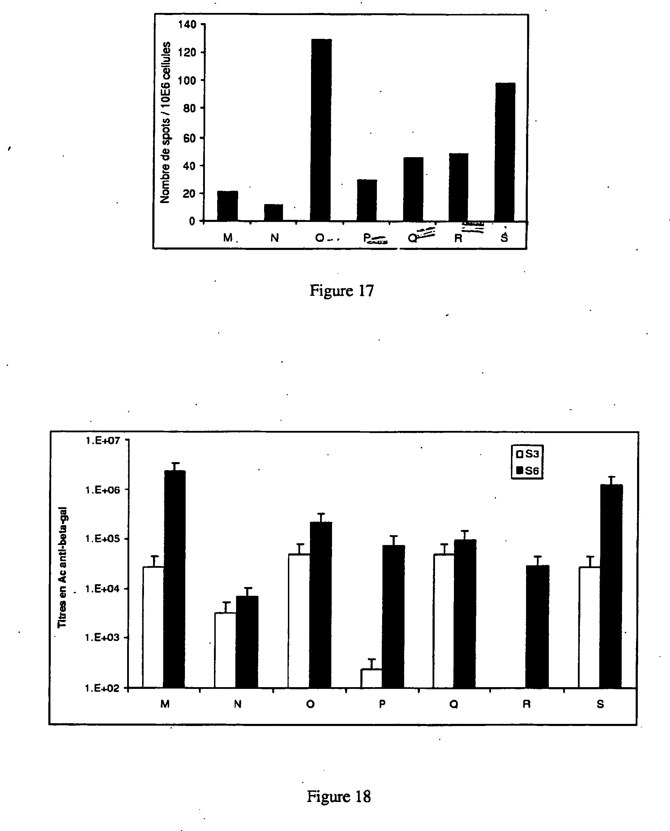

Les résultats sont présentés sur la figure 17 qui donne les réponses ELISPOT IFN-γ spécifiques du peptide beta-gal TPH. L'axe des ordonnées représente le nombre moyen de cellules sécrétant de l'IFN-γ de manière spécifique en réponse à une stimulation de 18h par le peptide beta-gal TPH sur un million de cellules stimulées. Cette réponse a été déterminée individuellement pour chaque souris. Le nombre présenté sur ce graphique correspond à la moyenne calculée à partir de toutes les souris d'un groupe (n=5).

Ces résultats mettent en évidence qu'il est possible de classer les combinaisons vaccinales en plusieurs catégories en fonction des réponses ELISPOT IFN-γ obtenues. Les meilleures réponses sont induites par les combinaisons PLA + MVA, en injection séparée (O) ou en deux injections simultanées (S). Des réponses intermédiaires sont obtenues avec une seule injection de PLA + MVA en même temps (groupes Q et R). Les réponses les plus faibles sont observées avec les groupes ne contenant qu'un seul immunogène PLA uniquement (M) ou MVA uniquement (N).

Ainsi, dans le modèle beta-galactosidase aussi, nous avons pu montrer que la combinaison vaccinale PLA + MVA était meilleure que la combinaison MVA + MVA pour induire une sécrétion d'IFN-γ spécifique du peptide TPH. De plus l'ordre d'injection semble aussi avoir une importance car parmi toutes les combinaisons PLA + MVA testées, c'est le groupe O, PLA puis MVA qui a permis d'obtenir le meilleur résultat. Il est à noter que c'est aussi la même combinaison qui s'était révélée la plus efficace dans le modèle p24 (exemple 1).

### 5.2. La réponse humorale spécifque de la protéine beta-gal

Les résultats sont présentés sur la figure 18 qui compare la cinétique d'apparition des réponses humorales spécifiques de la protéine beta-gal. L'axe des ordonnées représente le titre en IgG anti-beta-gal mesuré par ELISA de capture beta-gal ; l'échelle de cet axe est logarithmique. Le titre en anticorps a été déterminé individuellement pour chaque souris. Le nombre présenté sur ce graphique correspond à la moyenne calculée à partir de toutes les souris d'un groupe (n=5). S3 = semaine 3, S6 = semaine 6.

Ces résultats montrent que les meilleures réponses humorales sont obtenues pour le groupe PLA + PLA (M). Les titres induits chez les souris ayant reçues du PLA et du MVA à chaque injection (S) sont comparables. Dans les groupes O, P, Q et R, les titres sont plus bas d'environ un log. Parmi, toutes les combinaisons testées, le plus mauvais inducteur d'anticorps anti-beta-gal, c'est MVA + MVA. Dans le modèle beta-galactosidase, comme dans le modèle p24, toutes les combinaisons contenant du MVA et du PLA induisent un titre élevé, supérieur d'un log au titre induit par du MVA seul.

Comme dans l'exemple 1 (p24) changer une injection de PLA en MVA dans un schéma de vaccination ne nuit pas beaucoup aux titres en anticorps obtenus mais permet d'élargir les réponses cellulaires par rapport à celles qui sont générées uniquement par un vecteur PLA.

Même si l'immunisation PLA + MVA (S) ne permet pas d'induire la meilleure réponse immune selon chaque type de réponse analysée, dans l'ensemble c'est le groupe pour lequel on observe les réponses les plus larges et les plus intenses.

### Exemple 6: Immunisation de souris avec la combinaison vaccinale microparticules CPL/p24 et virus MVA gag

### 1. Modèle animal

Les expériences d'immunisation ont été réalisées chez des souris BALB/c (H-2^{d}) femelles âgées de 6 à 8 semaines au moment de la première immunisation.

### 2. Immunogènes administrés

Dans cette série d'expériences ont été utilisés seuls ou en combinaison les immunogènes suivants :
- les microparticules CPL utilisées dans ce travail sont formées par coprécipitation de deux polysaccharides ; le chitosane (Mahtani chitosan PVT Batch 124) et le sulfate de dextrane (Sigma : 31404). Les deux polymères sont au préalable mis en solution dans de l'eau à pH 4 contenant 50 mM de NaCl. Les solutions sont laissées sous agitation magnétique pendant 12 heures avant de préparer les particules. Ensuite, 20 ml de la solution de chitosane à 0,1% (masse molaire de 105000 g/mol et d'un degré d'acétylation 9) sont mélangés à 9,35 ml de la solution de sulfate de dextrane à 0,1%. La solution en défaut est versée rapidement sur la solution en excès placée sous agitation magnétique à 1000 rpm. Le mélange est laissé 30 secondes sur l'agitateur à 500 rpm, puis il est centrifugé pendant 30 minutes à 7 800 *g*. Le surnageant est éliminé par retournement, le culot est repris dans de l'eau puis à nouveau centrifugé pendant 30 minutes à 8 000 *g*. Les culots ainsi obtenus sont repris dans de l'eau et conservés à température ambiante sous agitation constante. Les particules obtenues sont caractérisées par une mesure de taille (granulomètre Coulter), et de charge (Zetasizer Malvem).
- Pour l'adsorption de la protéine p24 sur les CPL, la p24 à 1 g/l en tampon phosphate 10 mM pH 5,7 est mélangée volume pour volume avec des particules CPL à 1% de taux de solide soit 0,5% final. Les échantillons sont mis sous agitation sur une roue à température ambiante pendant 20 heures. Après incubation, les tubes sont récupérés et centrifugés 10 minutes à 1800 *g* à 20°C. Les surnageants sont récupérés et centrifugés 15 minutes à 3000 *g* à 20°C pour éliminer les particules pouvant rester en suspension, puis la concentration en protéine est déterminée en utilisant un dosage BCA (kit BCA Protein Assay, Pierce : 23225). Les culots sont repris dans 400 µl de tampon phosphate 10 mM. Les particules obtenues sont caractérisées par une mesure de taille et de charge.
- Le virus MVA gag (Modified Vaccinia Virus Ankara, virus de la vaccine atténué, souche Ankara) qui est recombinant pour le gène gag du virus VIH-1, codant pour la protéine p24. Le virus recombinant a été construit à Transgene, et produit et purifié comme décrit précédemment.

### 3. Immunisations

35 souris ont reçu 2 doses des immunogènes décrits dans le point 2 ci-dessus à 0 et 3 semaines. Les combinaisons vaccinales reçues par chaque groupe de souris sont indiquées dans le tableau 11 suivant. Toutes les injections ont été réalisées par voie sous-cutanée.

**Tableau 11**

| Code | Dose | Dose | Nombre de souris |
|---|---|---|---|
| | 1^{ère} injection | 2^{ème} injection | |
| | semaine 0 | semaine 3 | |
| T | CPL/p24 10 µg | CPL/p24 10 µg | 5 |
| U | MVA gag 10⁷ pfu | MVA gag 10⁷ pfu | 5 |
| V | CPL/p24 10 µg | MVA gag 10⁷ pfu | 5 |
| W | MVA gag 10 pfu | CPL/p24 10 µg | 5 |
| X | CPL/p24 10 µg + MVA gag 10⁷ pfu | | 5 |
| Y | | CPUp24 10 µg + MVA gag 10⁷ pfu | 5 |
| Z | CPL/p24 10 µg + MVA gag 10⁷ pfu | CPL/p24 10 µg + MVA gag 10⁷ pfu | 5 |

Pfu = plaque forming units, c'est l'unité de mesure de la dose de MVA utilisée. Le titre viral est exprimé en pfu selon la technique de dosage du stock viral utilisée.

Les animaux ont été sacrifiés 6 semaines après la première injection et le sang et la rate ont été prélevés pour les analyses immunologiques.

### 4. Analyses immunologiques

Réalisées suivant les mêmes protocoles que dans l'exemple 1.

### 5. Résultats

### 5.1. La réponse ELISPOT IFN-γ contre le peptide gag AMQ

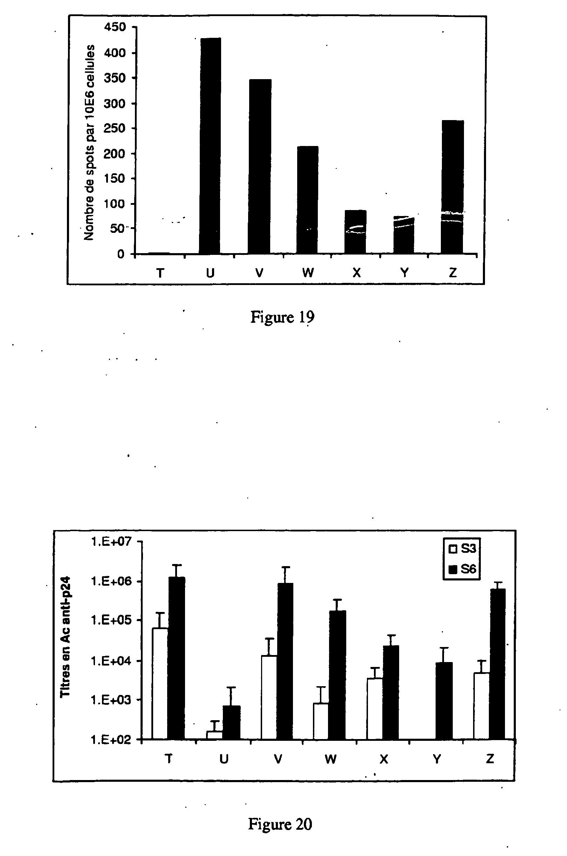

Les résultats sont présentés sur la figure 19 qui donne les réponses ELISPOT IFN-γ spécifiques du peptide p24 AMQ. L'axe des ordonnées représente le nombre moyen de cellules sécrétant de l'IFN-γ de manière spécifique en réponse à une stimulation de 18h par le peptide p24 AMQ sur un million de cellules stimulées. Cette réponse a été déterminée individuellement pour chaque souris. Le nombre présenté sur ce graphique correspond à la moyenne calculée à partir de toutes les souris d'un groupe (n=5).

Ces résultats montrent qu'il est possible de classer les combinaisons vaccinales en plusieurs catégories en fonction des réponses ELISPOT IFN-γ obtenues. La meilleure réponse est induite par la combinaison MVA + MVA (U). La deuxième catégorie contient les combinaisons CPL + MVA, en injection séparée (V) ou en deux injections simultanées (Z). Une seule injection simultanée de CPL et de MVA (X et Y) n'est pas assez efficace pour induire une réponse ELISPOT IFN-γ élevée. Finalement, la combinaison CPL + CPL (T) est la moins efficace.

Comme pour l'association PLA + MVA, l'association CPL + MVA est efficace pour induire une sécrétion d'IFN-γ spécifique du peptide AMQ. L'injection simultanée des deux immunogènes ne permet pas cependant d'améliorer les performances par rapport à administration séparée de 3 semaines (Z vs V).

### 5.2. La réponse humorale spécifique de la protéine p24

Les résultats sont présentés sur la figure 20 qui compare la cinétique d'apparition des réponses humorales spécifiques de la protéine p24. L'axe des ordonnées représente le titre en IgG anti-p24 mesuré par ELISA de capture p24 ; l'échelle de cet axe est logarithmique. Le titre en anticorps a été déterminé individuellement pour chaque souris. Le nombre présenté sur ce graphique correspond à la moyenne calculée à partir de toutes les souris d'un groupe (n=5). S3 = semaine 3, S6 = semaine 6.

Les résultats montrent que, pour les groupes ayant reçu 2 injections, toutes les combinaisons contenant du CPL et du MVA (V, W,Z) ou le CPL seul (T) induisent un titre élevé (10^{E}6), supérieur de 2-3 log au titre induit par du MVA seul. Cependant la combinaison CPL + MVA (V) est plus efficace que l'ordre d'injection inverse (W). Dans les groupes n'ayant reçu qu' une seule injection de CPL et MVA (X et Y), les titres en anticorps sont plus faibles, mais quand même supérieurs à ce que l'on obtient avec du MVA seul.

Comme pour l'association PLA + MVA, changer une injection de CPL en MVA dans un schéma de vaccination ne nuit pas aux titres en anticorps obtenus mais permet d'augmenter considérablement les réponses cellulaires par rapport à celles qui sont générées uniquement par un vecteur CPL.

### Conclusions :

L'ensemble de ces exemples nous a permis de montrer que la combinaison d'un vecteur synthétique colloïdal biorésorbable, tel que PLA, CPL, et d'un vecteur viral, tel que MVA et adénovirus, permet d'induire des réponses immunes efficaces et larges. De préférence, pour la meilleure efficacité, la combinaison devrait être administrée dans l'ordre vecteur synthétique, puis vecteur viral.

## Revendications

1. Composition pharmaceutique comprenant
- un vecteur synthétique colloïdal constitué de microparticules biorésorbables non toxiques comprenant un antigène protéique ou plusieurs antigènes protéiques, et
- un vecteur viral comprenant au moins une séquence nucléotidique codant pour un ou des antigènes protéique(s) compris dans le vecteur synthétique.

2. Composition selon la revendication 1, **caractérisée en ce que** les microparticules sont préparées à partir d'au moins un polymère choisi parmi les poly(acides α-hydroxylés).

3. Composition selon la revendication 1, **caractérisée en ce que** les microparticules sont préparées à partir d'au moins un polymère naturel.

4. Composition selon les revendications 1 à 3, **caractérisée en ce que** les microparticules comprennent des antigènes protéiques de types différents et constituant chacune un antigène associé à la même maladie.

5. Composition selon les revendications précédentes, **caractérisée en ce qu'**elle comprend, en tant que vecteur viral, un adénovirus ou un poxvirus.

6. Composition selon la revendication 5, **caractérisée en ce que** le poxvirus est le Modified Vaccinia Virus Ankara.

7. Composition selon l'une quelconque des revendications 1 à 6, **caractérisée en ce que** les antigènes protéiques sont d'origine virale.

8. Composition selon la revendication 7, **caractérisée en ce que** les antigènes protéiques d'origine virale sont des protéines du virus VIH.

9. Composition selon l'une quelconque des revendications 1 à 6, **caractérisée en ce que** l'antigène protéique est un antigène associé aux tumeurs.

10. Utilisation de la composition telle que définie dans les revendications 1 à 9 pour la préparation d'un vaccin prophylactique ou thérapeutique.

11. Utilisation de la composition selon la revendication 10 où le vaccin est pour administration dans le traitement ou la prévention des infections virales.

12. Utilisation de la composition selon la revendication 10 où le vaccin est pour administration dans le traitement ou la prévention d'un cancer.

13. Kit pharmaceutique comprenant la composition selon l'une quelconque des revendications 1 à 9.

14. Kit pharmaceutique selon la revendication 13, pour une administration simultanée, séparée ou échelonnée du vecteur synthétique et du vecteur viral.

15. Kit pharmaceutique selon la revendication 14 pour une administration échelonnée, le vecteur synthétique étant administré avant le vecteur viral.

## Claims

1. Pharmaceutical composition comprising
- a colloidal synthetic vector constituting of non-toxic bioresorbable microparticles comprising one proteic antigen or more, and
- a viral vector comprising at least one nucleotide sequence which codes for one or more protein antigen comprised in the synthetic vector.

2. Composition according to Claim 1, **characterised in that** the microparticles are prepared from at least one polymer chosen from amongst the poly(α-hydroxy acids).

3. Composition according to Claim 1, **characterised in that** the microparticles are prepared from at least one natural polymer.

4. Composition according to Claims 1 to 3, **characterised in that** the microparticles comprise protein antigen of different types, each constituting an antigen associated with the same disease.

5. Composition according to the preceding claims, **characterised in that** it comprises an adenovirus or a poxvirus as the viral vector.

6. Composition according to Claim 5, **characterised in that** the poxvirus is the Modified Vaccinia Virus Ankara.

7. Composition according to any one of Claims 1 to 6, **characterised in that** the protein antigen are of viral origin.

8. Composition according to Claim 7, **characterised in that** the protein antigen of viral origin are proteins of the HIV virus.

9. Composition according to any one of Claims 1 to 6, **characterised in that** the protein antigen is an antigen associated with tumours.

10. Use of the composition as defined in Claims 1 to 9 for the preparation of a prophylactic or therapeutic vaccine.

11. Use of the composition according to Claim 10, where the vaccine is for administration in the treatment or the prevention of viral infections.

12. Use of the composition according to Claim 10, where the vaccine is for administration in the treatment or the prevention of cancer.

13. Pharmaceutical kit comprising the composition according to Claims 1 to 9.

14. Pharmaceutical kit according to Claim 13 for a simultaneous administration, a separated administration or a staggered administration of the synthetic vector and of the viral vector.

15. Pharmaceutical kit according to Claim 14 for a staggered administration, the synthetic vector being administered before the viral vector.

## Patentansprüche

1. Pharmazeutische Zusammensetzung enthaltend
- einen kolloidalen synthetischen Vektor, der aus bioresorbierbaren, nicht toxischen Mikropartikeln gebildet ist, die ein oder mehrere Eiweißantigene enthalten und
- einen viralen Vektor, der mindestens eine Nucleotid-Sequenz, kodiert für ein oder mehrere Eiweißantigene, die in dem synthetischen Vektor enthalten sind.

2. Zusammensetzung nach Anspruch 1, **dadurch gekennzeichnet, dass** die Mikropartikel aus mindestens einem Polymer, ausgewählt aus Poly(α-Hydroxysäuren), hergestellt sind.

3. Zusammensetzung nach Anspruch 1, **dadurch gekennzeichnet, dass** die Mikropartikel aus mindestens einem natürlichen Polymer hergestellt sind.

4. Zusammensetzung nach den Ansprüchen 1 bis 3, **dadurch gekennzeichnet, dass** die Mikropartikel Eiweißantigene unterschiedlicher Arten enthalten und jedes ein derselben Krankheit zugeordnetes Antigen bildet.

5. Zuaammensetzung nach den vorhergehenden Ansprüchen, **dadurch gekennzeichnet, dass** sie, was den viralen Vektor betrifft, ein Adenovirus oder ein Poxvirus enthält.

6. Zusammensetzung nach Anspruch 5, **dadurch gekennzeichnet, dass** der Poxvirus ein modifizierter Vaccinia Virus Ankara ist.

7. Zusammensetzung nach einem beliebigen der Ansprüche 1 bis 6, **dadurch gekennzeichnet, dass** die Eiweißantigene viralen Ursprungs sind.

8. Zusammensetzung nach Anspruch 7, **dadurch gekennzeichnet, dass** die Eiweißantigene viralen Ursprungs Proteine des Virus VIH sind.

9. Zusammensetzung nach einem beliebigen der Ansprüche 1 bis 6, **dadurch gekennzeichnet, dass** das Eiweißantigen ein den Tumoren zugeordnetes Antigen ist.

10. Verwendung der Zusammensetzung, wie in den Ansprüchen 1 bis 9 definiert, für die Herstellung eines prephylaktischen oder therapeutischen Impfstoffs.

11. Verwendung der Zusammensetzung nach Anspruch 10, bei der der Impfstoff zur Applikation bei der Behandlung oder der Prävention von viralen Infekten dient.

12. Verwendung der Zusammensetzung nach Anspruch 10, bei der der Impfstoff zur Applikation in der Behandlung oder Prävention von Krebs dient.

13. Pharmazeutisches Kit, die Zusammensetzung nach einem beliebigen der Ansprüche 1 bis 9 umfassend.

14. Pharmazeutisches Kit nach Anspruch 13 für eine gleichzeitige, getrennte oder gestufte Applikation des synthetischen Vektors und des viralen Vektors.

15. Pharmazeutisches Kit nach Anspruch 14 für eine gestufte Applikation, wobei der synthetische Vektor vor dem viralen Vektor verabreicht wird.


### SEQUENCE LISTING

<110> bioMérieux
   Centre National de la Recherche Scientifique
<120> Composition comprenant un vecteur synthétique colloïdal biorésorbable et un vecteur viral et son utilisation prophylactique, thérapeutique et diagnostique
<130> Primeboost
<160> 3
<170> PatentIn version 3.3
<210> 1
   <211> 432
   <212> PRT
   <213> Human immunodeficiency virus
<400> 1
   Met Ala Ala Arg Ala Ser Ile Leu Ser Gly Gly Glu Leu Asp Arg Trp
   1               5                   10                  15
   Glu Lys Ile Arg Leu Arg Pro Gly Gly Lys Lys Arg Tyr Lys Leu Lys
               20                  25                  30
   His Ile Val Trp Ala Ser Arg Glu Leu Glu Arg Phe Ala Val Asn Pro
           35                  40                  45
   Gly Leu Leu Glu Thr Ser Glu Gly Cys Arg Gln Ile Leu Gly Gln Leu
       50                  55                  60
   Gln Pro Ala Ile Gln Thr Gly Ser Glu Glu Leu Lys Ser Leu Tyr Asn
   65                  70                  75                  80
   Thr Val Ala Thr Leu Tyr Cys Val His Gln Arg Ile Glu Val Lys Asp
                   85                  90                  95
   Thr Lys Glu Ala Leu Asp Arg Ile Glu Glu Glu Gln Asn Lys Ser Lys
               100                 105                 110
   Gln Lys Ala Gln Pro Ala Ala Ala Asp Lys Gly Asn Ser Ser Gln Ala
           115                 120                 125
   Ser Gln Asn Tyr Pro Ile Val Gln Asn Ile Gln Gly Gln Met Val His
       130                 135                 140
   Gln Ala Ile Ser Pro Arg Thr Leu Asn Ala Trp Val Lys Val Val Glu
   145                 150                 155                 160
   Glu Lys Ala Phe Ser Pro Glu Val Ile Pro Met Phe Ser Ala Leu Ser
                   165                 170                 175
   Glu Gly Ala Thr Pro Gln Asp Leu Asn Thr Met Leu Asn Thr Val Gly
               180                 185                 190
   Gly His Gln Ala Ala Met Gln Met Leu Lys Glu Thr Ile Asn Glu Glu
           195                 200                 205
   Ala Ala Glu Trp Asp Arg Leu His Pro Val His Ala Gly Pro Ile Ala
       210                 215                 220
   Pro Gly Gln Met Arg Glu Pro Arg Gly Ser Asp Ile Ala Gly Thr Thr
   225                 230                 235                 240
   Ser Thr Leu Gln Glu Gln Ile Gly Trp Met Thr Asn Asn Pro Pro Ile
                   245                 250                 255
   Pro Val Gly Glu Ile Tyr Lys Arg Trp Ile Ile Leu Gly Leu Asn Lys
               260                 265                 270
   Ile Val Arg Met Tyr Ser Pro Thr Ser Ile Leu Asp Ile Arg Gln Gly
           275                 280                 285
   Pro Lys Glu Pro Phe Arg Asp Tyr Val Asp Arg Phe Tyr Lys Thr Leu
       290                 295                 300
   Arg Ala Glu Gln Ala Ser Gln Glu Val Lys Asn Trp Met Thr Glu Thr
   305                 310                 315                 320
   Leu Leu Val Gln Asn Ala Asn Pro Asp Cys Lys Thr Ile Leu Lys Ala
                   325                 330                 335
   Leu Gly Pro Ala Ala Thr Leu Glu Glu Met Met Thr Ala Cys Gln Gly
               340                 345                 350
   Val Gly Gly Pro Gly His Lys Ala Arg Ile Leu Ala Glu Ala Met Ser
           355                 360                 365
   Gln Val Thr Asn Ser Ala Thr Ile Met Met Gln Arg Gly Asn Phe Arg
       370                 375                 380
   Asn Gln Arg Lys Ile Val Lys Cys Phe Asn Cys Gly Lys Glu Gly His
   385                 390                 395                 400
   Ile Ala Arg Asn Cys Arg Ala Pro Arg Lys Lys Gly Cys Trp Lys Cys
                   405                 410                 415
   Gly Lys Glu Gly His Gln Met Lys Asp Cys Thr Glu Arg Gln Ala Asn
               420                 425                 430
<210> 2
   <211> 9
   <212> PRT
   <213> Human immunodeficiency virus
<400> 2
   Ala Met Gln Met Leu Lys Glu Thr Ile
   1               5
<210> 3
   <211> 9
   <212> PRT
   <213> Human immunodeficiency virus
<400> 3
   Thr Pro His Pro Ala Arg Ile Gly Leu
   1               5